(19) Europäisches Patentamt
European Patent Office
Office européen des brevets

(11) Veröffentlichungsnummer: **0 304 552**
**A1**

# (12) EUROPÄISCHE PATENTANMELDUNG

(21) Anmeldenummer: 88108238.2

(22) Anmeldetag: 24.05.88

(51) Int. Cl.[4]: C07D 409/06 , C07D 405/06 , C07D 403/06 , C07D 401/06 , C07D 417/06 , C07D 249/08 , C07D 233/60 , A01N 43/653 , A01N 43/50

(30) Priorität: 03.06.87 DE 3718562
23.04.88 DE 3813841

(43) Veröffentlichungstag der Anmeldung:
01.03.89 Patentblatt 89/09

(84) Benannte Vertragsstaaten:
BE CH DE FR GB IT LI NL

(71) Anmelder: BAYER AG
Konzernverwaltung RP Patentabteilung
D-5090 Leverkusen 1 Bayerwerk(DE)

(72) Erfinder: Jautelat, Manfred, Dr.
Muellersbaum 28
D-5093 Burscheid(DE)
Erfinder: Dutzmann, Stefan, Dr.
Leinenweberweg 33
D-4000 Düsseldorf 13(DE)

## (54) Heterocyclische Hydroxyethylazole.

(57) Neue heterocyclische Hydroxyethyl- azole der Formel

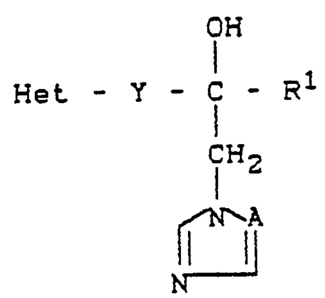

(I)

in welcher
A für ein Stickstoffatom oder eine CH-Gruppe steht,
Y für die Gruppierungen $-CH_2-CH_2-$, $-CH=CH-$ oder $-C\equiv C-$ steht,
$R^1$ für gegebenenfalls substituiertes Alkyl, gegebenenfalls substituiertes Alkenyl, gegebenenfalls substituiertes Cycloalkyl oder für gegebenenfalls substituiertes Aryl steht und
Het für einen gegebenenfalls substituierten und gegebenenfalls benzanellierten, fünf- oder sechsgliedrigen heterocyclischen Rest mit bis zu 3 Heteroatomen steht,
sowie deren Säureadditions-Salze und Metallsalzkomplexe,
Verfahren zur Herstellung der neuen Stoffe und deren Verwendung als Fungizide und Pflanzenwachstumsregulatoren.
Neue Alkinole der Formel

EP 0 304 552 A1

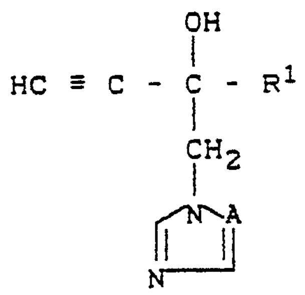

(II)

in welcher

A und $R^1$ die oben angegebene Bedeutung haben,

Verfahren zu deren Herstellung und deren Verwendung als Zwischenprodukte zur Synthese von heterocyclischen Hydroxyethyl-azolen der Formel (I).

# Heterocyclische Hydroxyethylazole

Die vorliegende Erfindung betrifft neue heterocyclische Hydroxyethylazole, Verfahren zu deren Herstellung und deren Verwendung als Fungizide und Pflanzenwachstumsregulatoren.

Es sind bereits zahlreiche Hydroxyethyl-azol-Derivate mit fungiziden und pflanzenwuchsregulierenden Eigenschaften bekannt geworden. So lassen sich zum Beispiel substituierte Phenylethyl-1-hydroxyethyl-azol-Derivate zur Bekämpfung von Pilzen und zur Regulierung des Pflanzenwachstums verwenden (vgl. EP-OS 0 040 345). Die Wirksamkeit dieser Stoffe ist gut. Bei niedrigen Aufwandmengen läßt die fungizide und pflanzenwuchsregulierende Aktivität in manchen Fällen allerdings zu wünschen übrig.

Weiterhin ist bekannt, daß auch bestimmte heterocyclische Hydroxyethyl-azole zur Bekämpfung von Pilzen und zur Regulierung des Pflanzenwachstums eingesetzt werden können (vgl. EP-OS 0 207 590 und EP-OS 0 212 841). Auch die Wirksamkeit dieser Stoffe ist aber nicht immer ausreichend.

Es wurden nun neue heterocyclische Hydroxyethylazole der Formel

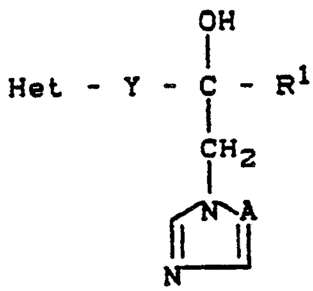

(I)

in welcher

A für ein Stickstoffatom oder eine CH-Gruppe steht,

Y für die Gruppierungen $-CH_2-CH_2-$, $-CH=CH-$ oder $-C\equiv C-$steht,

$R^1$ für gegebenenfalls substituiertes Alkyl, gegebenenfalls substituiertes Alkenyl, gegebenenfalls substituiertes Cycloalkyl oder für gegebenenfalls substituiertes Aryl steht und

Het für einen gegebenenfalls substituierten und gegebenenfalls benzanellierten, fünf- oder sechsgliedrigen heterocyclischen Rest mit bis zu 3 Heteroatomen steht,

sowie deren Säureadditions-Salze und Metallsalz-Komplexe gefunden.

Die Verbindungen der Formel (I) enthalten ein asymmetrisch substituiertes Kohlenstoffatom und können deshalb in den beiden optischen Isomerenformen anfallen. Wenn Y für die Gruppierung $-CH=CH-$ steht, können die Verbindungen der Formel (I) zusätzlich in zwei geometrischen Isomerenformen vorliegen. Die vorliegende Erfindung betrifft sowohl die Isomerengemische als auch die einzelnen Isomeren.

Weiterhin wurde gefunden, daß man heterocyclische Hydroxyethylazole der Formel (I) sowie deren Säureadditions-Salze und Metallsalz-Komplexe erhält, wenn man

a) Alkinole der Formel

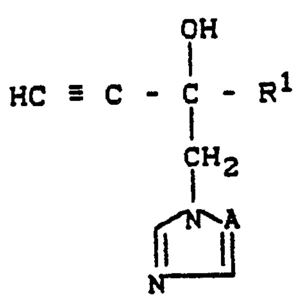

(II)

in welcher

A und $R^1$ die oben angegebene Bedeutung haben,

mit Halogen-Heterocyclen der Formel

Het - X (III)

in welcher

Het die oben angegebene Bedeutung hat und
X für Chlor, Brom oder Jod steht,
in Gegenwart eines Säurebindemittels und in Gegenwart eines Katalysators sowie in Gegenwart eines Verdünnungsmittels umsetzt,
oder

b) heterocyclische Hydroxyethyl-azole der Formel

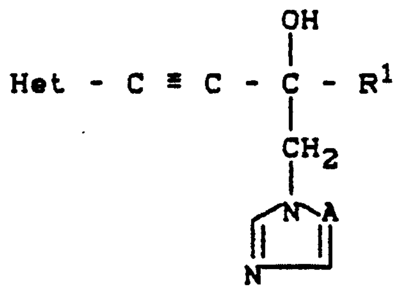

(Ia)

in welcher
Het, A und $R^1$ die oben angegebene Bedeutung haben,
entweder
α) mit Wasserstoff in Gegenwart eines Hydrierkatalysators und in Gegenwart eines Verdünnungsmittels umsetzt,
oder
β) mit Wasserstoffdonatoren in Gegenwart eines Verdünnungsmittels umsetzt,
und gegebenenfalls halogeniert und gegebenenfalls anschließend an die so erhaltenen Verbindungen der Formel (I) eine Säure oder ein Metallsalz addiert.

Außerdem wurde gefunden, daß die neuen heterocyclischen Hydroxyethylazole der Formel (I) sowie deren Säureadditions-Salze und Metallsalz-Komplexe sehr gute fungizide und pflanzenwuchsregulierende Eigenschaften aufweisen.

Überraschenderweise besitzen die erfindungsgemäßen Wirkstoffe eine deutlich bessere fungizide und pflanzenwuchsregulierende Wirksamkeit als die konstitutionell ähnlichsten Stoffe, die aus dem Stand der Technik bekannt sind.

Die erfindungsgemäßen heterocyclischen Hydroxyethylazole sind durch die Formel (I) allgemein definiert. In dieser Formel stehen vorzugsweise

A für ein Stickstoffatom oder eine CH-Gruppe,
Y für die Gruppierungen $-CH_2-CH_2-$, $-CH=CH-$ oder $-C\equiv C-$,
$R^1$ für geradkettiges oder verzweigtes Alkyl mit 3 bis 7 Kohlenstoffatomen, wobei jeder dieser Reste substituiert sein kann durch Halogen, Cycloalkyl mit 3 bis 7 Kohlenstoffatomen, Phenyl und/oder Halogenphenyl, für gegebenenfalls durch Halogen, Phenyl und/oder Halogenphenyl substituiertes Alkenyl mit 3 bis 6 Kohlenstoffatomen, ferner für Cycloalkyl mit 3 bis 7 Kohlenstofatomen, wobei jeder dieser Reste durch Alkyl mit 1 bis 4 Kohlenstoffatomen substituiert sein kann, oder für Phenyl, das einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano, und
Het für einen gegebenenfalls benzanellierten fünfgliedrigen Heterocyclus mit 1 bis 3 Stickstoffatomen, für einen gegebenenfalls benzanellierten fünfgliedrigen Heterocyclus mit einem Sauerstoffatom, für einen gegebenenfalls benzanellierten fünfgliedrigen Heterocyclus mit einem Schwefelatom, für einen gegebenenfalls benzanellierten fünfgliedrgen Heterocyclus mit einem Sauerstoffatom und einem Schwefelatom, für einen gegebenenfalls benzanellierten fünfgliedrigen Heterocyclus mit einem Sauerstoffatom und einem Stickstoffatom, für einen gegebenenfalls benzanellierten fünfgliedrigen Heterocyclus mit einem Schwefelatom und einem Stickstoffatom, für einen gegebenenfalls benzanellierten sechsgliedrigen Heterocyclus mit 1 bis 3 Stickstoffatomen, wobei jeder der zuvor genannten heterocyclischen Reste ein- oder mehrfach, gleichartig oder verschieden substituiert sein kann durch Halogen, Alkyl mit 1 bis 4 Kohlenstoffatomen,

Hydroxyalkyl mit 1 bis 4 Kohlenstoffatomen, Hydroxyalkinyl mit 3 bis 8 Kohlenstoffatomen, Alkoxy mit 1 oder 2 Kohlenstoffatomen, Alkylthio mit 1 oder 2 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, wie Fluor- oder Chloratomen, Formyl, Dialkoxymethyl mit 1 oder 2 Kohlenstoffatomen in jeder Alkoxygruppe, Acyl mit 2 bis 4 Kohlenstoffatomen, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 3 Kohlenstoffatomen im Alkylteil, Nitro und/oder Cyano oder durch den Rest der Formel

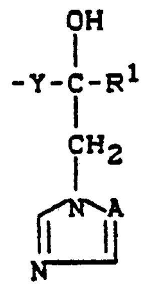

wobei $R^1$, A und Y die oben angegebenen Bedeutungen haben.

Besonders bevorzugt sind diejenigen Verbindungen der Formel (I), in denen
A für ein Stickstoffatom oder eine CH-Gruppe steht,
Y für die Gruppierungen $-CH_2-CH_2-$, $-CH=CH-$ oder $-C\equiv C-$steht,
$R^1$ für Isopropyl, tert.-Butyl, tert.-Pentyl, 1-Ethyl-1-methyl-propyl, 1,1-Dimethyl-pentyl, 1,1,2-Trimethylpropyl oder 1,1 Dimethyl-prop-2-enyl steht, wobei jeder dieser zuvorgenannten Reste substituiert sein kann durch Fluor, Chlor, Brom, Phenyl, Chlorphenyl, Dichlorphenyl, Fluorphenyl und/oder Difluorphenyl,
ferner für 1-Methyl-cyclohexyl, Cyclohexyl, 1-Methylcyclopropyl, Cyclopropyl, 1-Methyl-cyclopentyl, Cyclopentyl oder 1-Ethyl-cyclopentyl steht, oder
für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Methoxycarbonyl, Ethoxycarbonyl, Methoximinomethyl, 1-Methoximinoethyl, Nitro und/oder Cyano
und
Het für Pyrazolyl, Imidazolyl, 1,2,4-Triazolyl, Pyrrolyl, Furanyl, Thienyl, Thiazolyl, Oxazolyl, Pyridinyl, Pyrimidinyl, Triazinyl, Chinolinyl, Isochinolinyl, Chinazolinyl, Indolyl, Benzothienyl, Benzofuranyl, Benzothiazolyl oder Benzoimidazolyl steht, wobei jeder dieser Reste einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Hydroxymethyl, Hydroxyethyl, Hydroxyalkinyl mit 4 bis 6 Kohlenstoffatomen, Methoxycarbonyl, Ethoxycarbonyl, Methoximinomethyl, 1-Methoximino-ethyl, Nitro, Cyano, Formyl, Dimethoxymethyl, Acetyl und/oder Propionyl oder durch den Rest der Formel

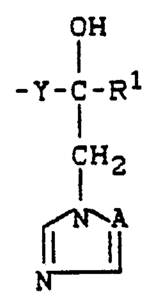

wobei $R^1$, A und Y die oben genannten Bedeutungen haben.

Eine Gruppe ganz besonders bevorzugter Verbindungen sind diejenigen Stoffe der Formel (I), in denen
A für ein Stickstoffatom steht,
Y für die Gruppierungen $-CH_2-CH_2-$, $-CH=CH$ oder $-C\equiv C-$steht,
$R^1$ für tert.-Butyl, tert.-Pentyl, 1-Ethyl-1-methylpropyl, 1,1,2-Trimethylpropyl, 1,1-Dimethyl-pentyl oder 1,1-Dimethyl-prop-2-enyl steht, wobei jeder dieser Reste ein- oder zweifach durch Fluor und/oder Chlor, durch

Phenyl, Chlorphenyl und/oder Fluorphenyl substituiert sein kann, ferner für 1-Methyl-cyclohexyl, 1-Methyl-cyclopentyl oder 1-Ethyl-cyclopentyl steht, oder für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Nitro, Cyano und/oder Methoximinomethyl,

und

Het für Furanyl, Thienyl, Thiazolyl, Pyridinyl, Pyrimidinyl, Chinolinyl, Benzothienyl, Benzothiazolyl oder Benzofuranyl steht, wobei jeder dieser Reste einfach bis dreifach, gleichartig oder verschieden substituiert sein kann durch Fluor, Chlor, Brom, Methyl, Ethyl, Methoxy, Ethoxy, Methylthio, Trifluormethyl, Trifluormethoxy, Trifluormethylthio, Hydroxymethyl, 3-Hydroxy-3-methylbut-1-in-1-yl, Methoxycarbonyl, Ethoxycarbonyl, Formyl, Dimethoxymethyl, Methoximinomethyl, Methoximino-ethyl, Nitro und/oder Cyano oder durch den Rest der Formel

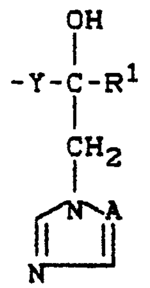

wobei $R^1$, A und Y die oben genannten Bedeutungen haben.

Bevorzugte erfindungsgemäße Verbindungen sind auch Additionsprodukte aus Säuren und denjenigen heterocyclischen Hydroxyethylazolen der Formel (I), in denen A, Y, $R^1$ und Het die Bedeutungen haben, die bereits vorzugsweise für diese Substituenten genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie z.B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen heterocyclischen Hydroxyethylazolen der Formel (I), in denen A, Y, $R^1$ und Het die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phophorsäure, Salpetersäure und Schwefelsäure.

Als spezielle Beispiele für erfindungsgemäße Stoffe seien die in der folgenden Tabelle aufgeführten heterocyclischen Hydroxyethylazole genannt.

Tabelle 1:

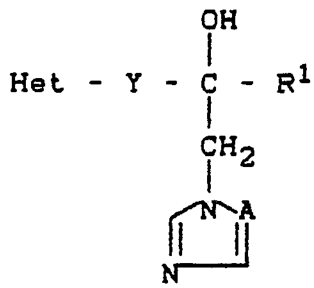

(I)

| Het | Y | $R^1$ | A |
|---|---|---|---|
| 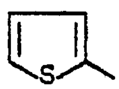 | $-CH_2-CH_2-$ | 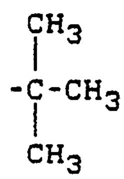 | N |
| " | " | 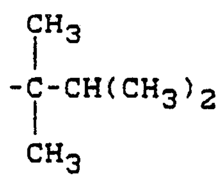 | N |
| " | " | 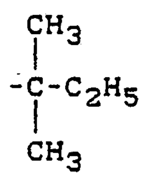 | N |
| " | " | 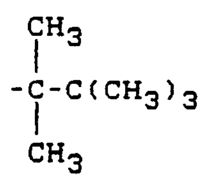 | N |

<u>Tabelle 1</u> (Fortsetzung)

| Het | Y | $R^1$ | A |
|---|---|---|---|
| 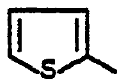 | $-CH_2-CH_2-$ | 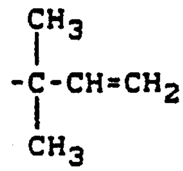 | N |
| " | " | 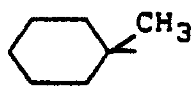 | N |
| " | " | 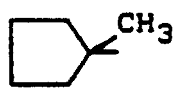 | N |
| " | " | 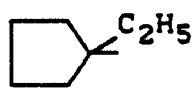 | N |
| " | " | 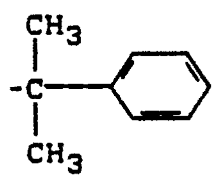 | N |
| " | " | 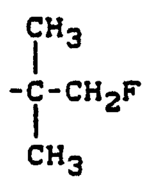 | N |
| " | " | 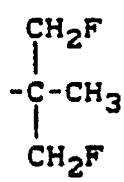 | N |

Tabelle 1 (Fortsetzung)

| Het | Y | $R^1$ | A |
|---|---|---|---|
| 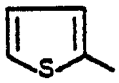 | $-CH_2-CH_2-$ | 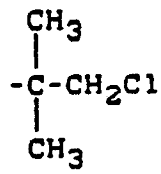 | N |
| " | " | 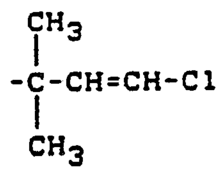 | N |
| " | " | 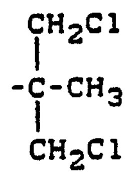 | N |
| " | " | 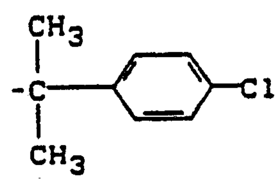 | N |
| " | " | 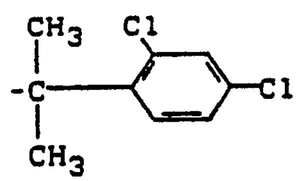 | N |
| " | " | 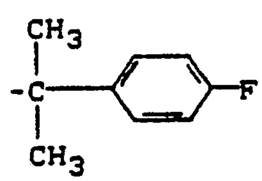 | N |

Tabelle 1 (Fortsetzung)

| Het | Y | $R^1$ | A |
|---|---|---|---|
| 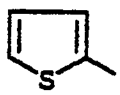 | $-CH_2-CH_2-$ | 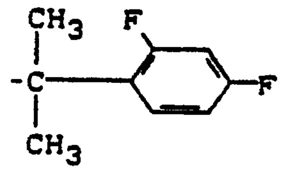 | N |
| " | " | 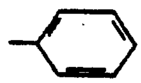 | N |
| " | " | 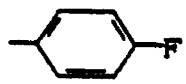 | N |
| " | " | 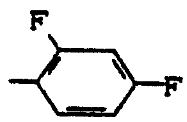 | N |
| " | " | 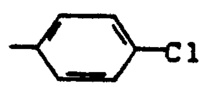 | N |
| " | " | 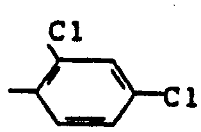 | N |

Tabelle 1 (Fortsetzung)

| Het | Y | $R^1$ | A |
|---|---|---|---|
| 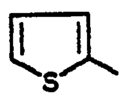 | $-CH_2-CH_2-$ | 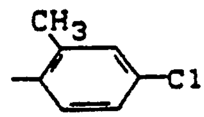 | N |
| " | " | 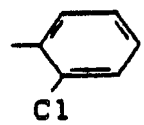 | N |
| " | " | 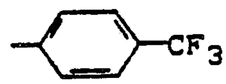 | N |
| " | " | 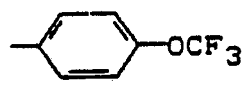 | N |
| " | " | 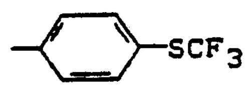 | N |
| " | " | 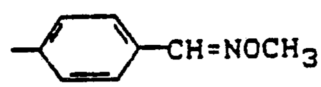 | N |
| " | " | 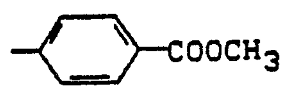 | N |

**Tabelle 1** (Fortsetzung)

| Het | Y | $R^1$ | A |
|---|---|---|---|
| 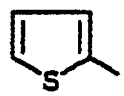 | $-CH_2-CH_2-$ | 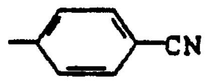 | N |
| " | " | 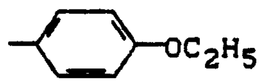 | N |
| " | $-CH=CH-$ | 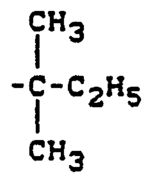 | N |
| " | " | 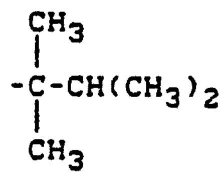 | N |
| " | " | 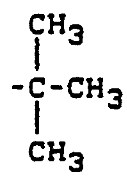 | N |
| " | " | 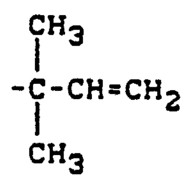 | N |

Tabelle 1 (Fortsetzung)

| Het | Y | $R^1$ | A |
|---|---|---|---|
| 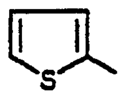 | -CH=CH- | 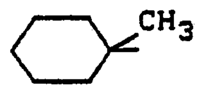 | N |
| " | " | 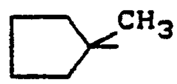 | N |
| " | " | 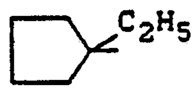 | N |
| " | " | 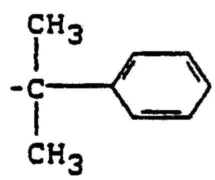 | N |
| " | " | 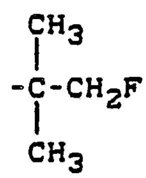 | N |
| " | " | 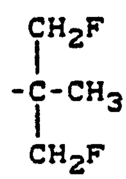 | N |

**Tabelle 1** (Fortsetzung)

| Het | Y | $R^1$ | A |
|---|---|---|---|
| 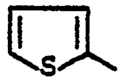 | -CH=CH- | 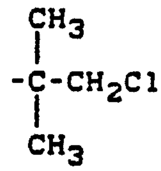 | N |
| " | " | 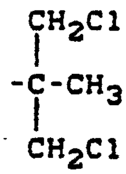 | N |
| " | " | 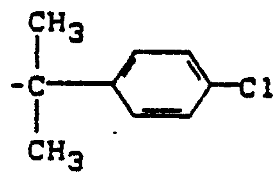 | N |
| " | " | 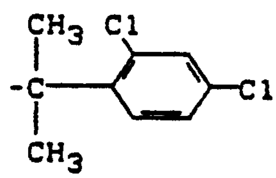 | N |
| " | " | 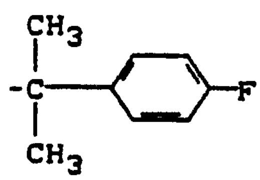 | N |

Tabelle 1 (Fortsetzung)

| Het | Y | $R^1$ | A |
|---|---|---|---|
| 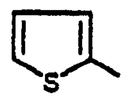 | -CH=CH- | 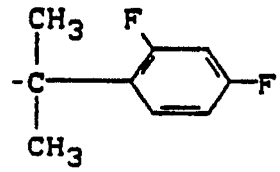 | N |
| " | " | 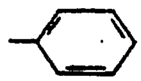 | N |
| " | " | 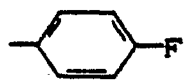 | N |
| " | " | 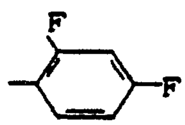 | N |
| " | " | 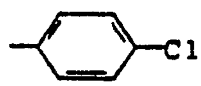 | N |
| " | " | 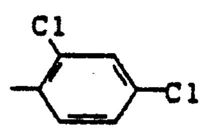 | N |

**Tabelle 1** (Fortsetzung)

| Het | Y | $R^1$ | A |
|---|---|---|---|
| 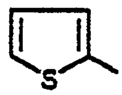 | -CH=CH- | 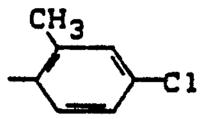 | N |
| " | " | 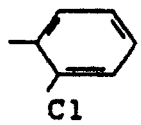 | N |
| " | " | 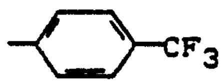 | N |
| " | " | 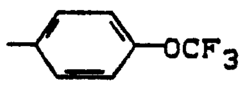 | N |
| " | " | 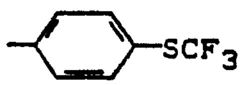 | N |
| " | " | 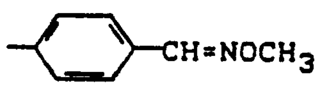 | N |
| " | " | 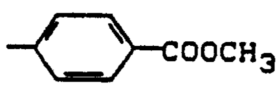 | N |

Tabelle 1 (Fortsetzung)

| Het | Y | $R^1$ | A |
|---|---|---|---|
| 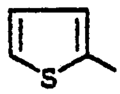 | -CH=CH- | 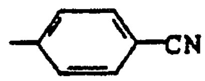 | N |
| " | " | 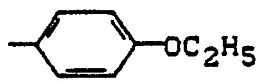 | N |
| 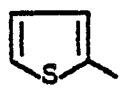 | -C≡C- | 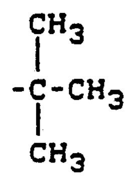 | N |
| " | " | 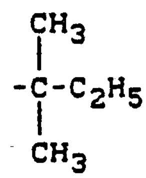 | N |
| " | " | 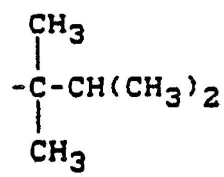 | N |
| " | " | 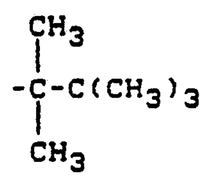 | N |

Tabelle 1 (Fortsetzung)

| Het | Y | $R^1$ | A |
|---|---|---|---|
| 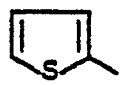 | -C≡C- | 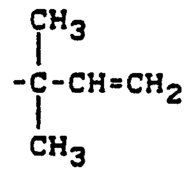 | N |
| " | " | 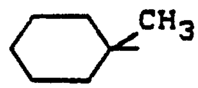 | N |
| " | " | 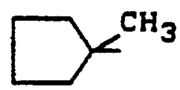 | N |
| " | " | 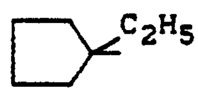 | N |
| " | " | 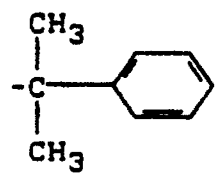 | N |
| " | " | 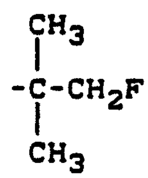 | N |

Tabelle 1 (Fortsetzung)

| Het | Y | $R^1$ | A |
|---|---|---|---|
| 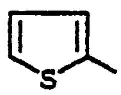 | $-C\equiv C-$ | 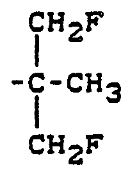 | N |
| " | " | 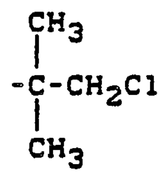 | N |
| " | " | 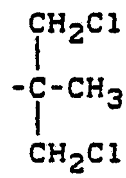 | N |
| " | " | 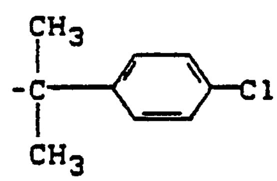 | N |
| " | " | 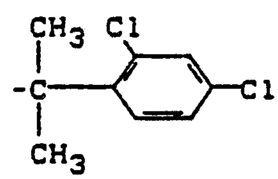 | N |
| " | " | 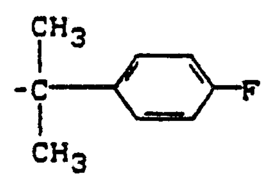 | N |

<u>Tabelle 1</u> (Fortsetzung)

| Het | Y | R[1] | A |
|---|---|---|---|
| 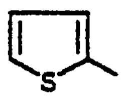 | $-C\equiv C-$ | 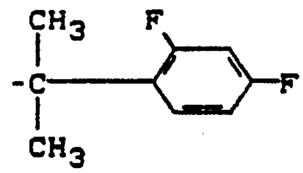 | N |
| " | " | 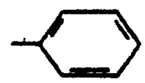 | N |
| " | " | 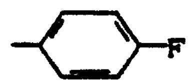 | N |
| " | " | 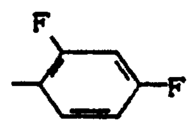 | N |
| " | " | 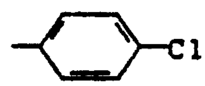 | N |
| " | " | 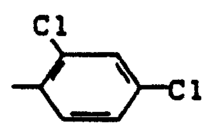 | N |
| " | " | 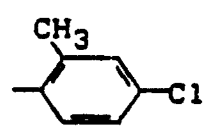 | N |

Tabelle 1 (Fortsetzung)

| Het | Y | $R^1$ | A |
|---|---|---|---|
| 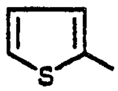 | -C≡C- | 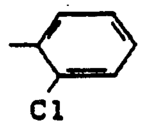 | N |
| " | " | 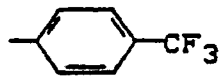 | N |
| " | " | 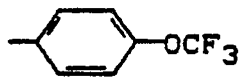 | N |
| " | " | 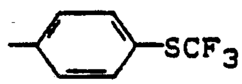 | N |
| " | " | 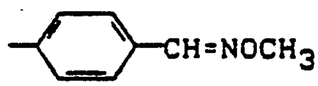 | N |
| " | " | 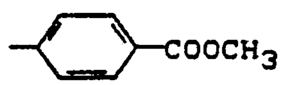 | N |
| " | " | 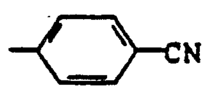 | N |
| " | " | 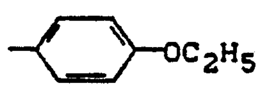 | N |

**Tabelle 1** (Fortsetzung)

| Het | Y | $R^1$ | A |
|---|---|---|---|
| 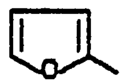 | $-CH_2-CH_2-$ | $-C(CH_3)_3$ | N |
| 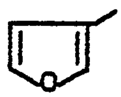 | " | " | N |
| 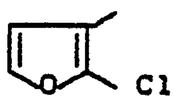 | " | " | N |
| 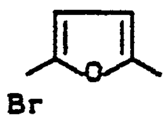 | " | " | N |
| 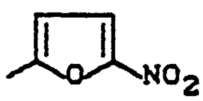 | " | " | N |
| 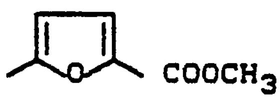 | " | $CH_3$<br>$-C-CH_2Cl$<br>$CH_3$ | N |
| 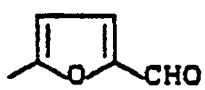 | " | $-C(CH_3)_3$ | N |

Tabelle 1 (Fortsetzung)

| Het | Y | $R^1$ | A |
|---|---|---|---|
| 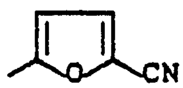 | $-CH_2-CH_2-$ | $-C(CH_3)_3$ | N |
| 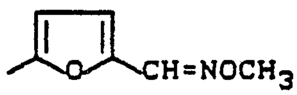 | " | " | N |
| 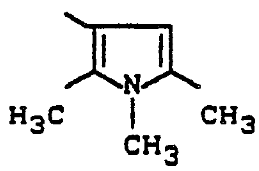 | " | " | N |
| 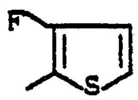 | " | " | N |
| 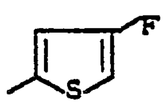 | " | " | N |
| 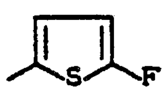 | " | " | N |
| 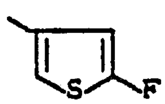 | " | " | N |

<u>Tabelle 1</u> (Fortsetzung)

| Het | Y | $R^1$ | A |
|---|---|---|---|
| 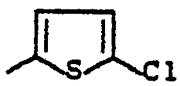 | $-CH_2-CH_2-$ | $-C(CH_3)_3$ | N |
| 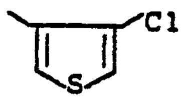 | " | " | N |
| 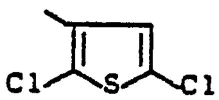 | " | " | N |
| 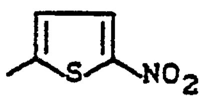 | " | " | N |
| 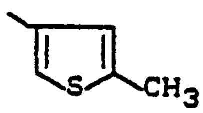 | " | " | N |
| 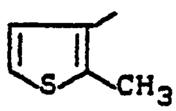 | " | " | N |
| 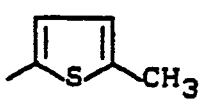 | " | " | N |
| 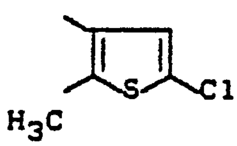 | " | " | N |

Tabelle 1 (Fortsetzung)

| Het | Y | $R^1$ | A |
|---|---|---|---|
| 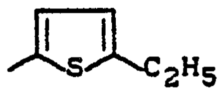 | $-CH_2-CH_2-$ | $-C(CH_3)_3$ | N |
| 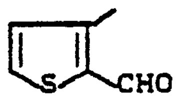 | " | " | N |
| 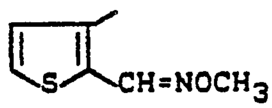 | " | " | N |
| 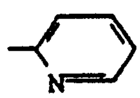 | $-CH=CH-$ | " | N |
| 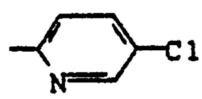 | $-CH_2-CH_2-$ | " | N |
| 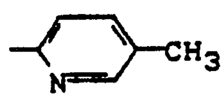 | " | " | N |
| 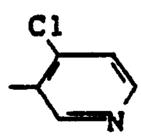 | " | " | N |

<u>Tabelle 1</u> (Fortsetzung)

| Het | Y | $R^1$ | A |
|---|---|---|---|
| 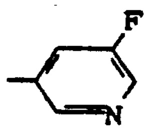 | $-CH_2-CH_2-$ | $-C(CH_3)_3$ | N |
| 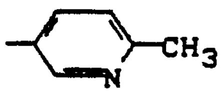 | " | " | N |
| 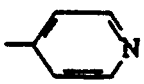 | " | " | N |
| 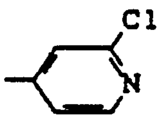 | " | " | N |
| 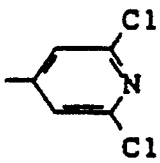 | " | " | N |
| 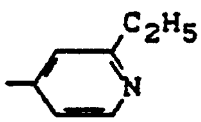 | " | " | N |

Tabelle 1 (Fortsetzung)

| Het | Y | $R^1$ | A |
|---|---|---|---|
| 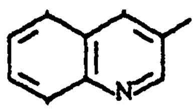 | $-CH_2-CH_2-$ | $-C(CH_3)$ | N |
| 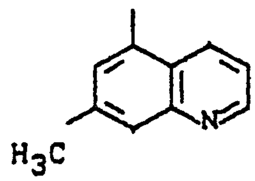 | " | " | N |
| 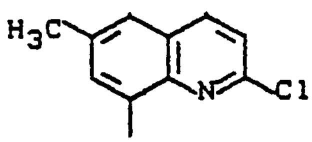 | " | " | N |
| 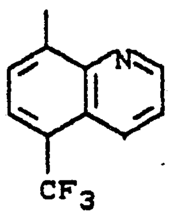 | " | " | N |
| 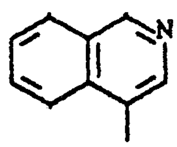 | " | " | N |
| 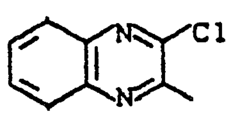 | " | " | N |

Verwendet man 4,4-Dimethyl-3-hydroxy-3-[(1,2,4-triazol-1-yl)-methyl]-1-pentin und 3-Bromthiophen als Ausgangsstoffe und Bistriphenylphosphin-palladiumdichlorid und Kupfer(I)-iodid als Katalysator, so kann der Verlauf des erfindungsgemäßen Verfahrens (a) durch das folgende Formelschema wiedergegeben werden:

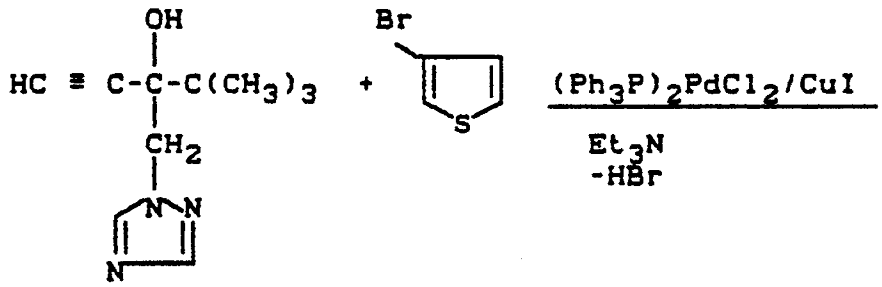


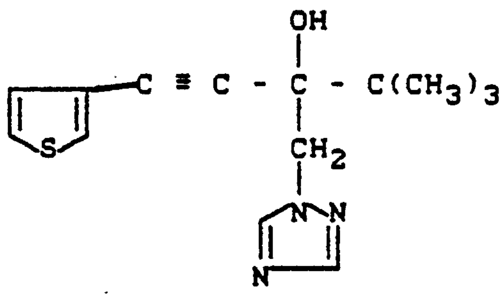

(I)

Verwendet man 4,4-Dimethyl-3-hydroxy-1-(3-thienyl)-3-[(1,2,4-triazol-1-yl)-methyl]-1-pentin als Ausgangsstoff, Wasserstoff als Hydrierungsmittel und Palladium auf Aktivkohle als Katalysator, so kann der Verlauf des erfindungsgemäßen Verfahrens (b, Variante α) durch das folgende Formelschema wiedergegeben werden:

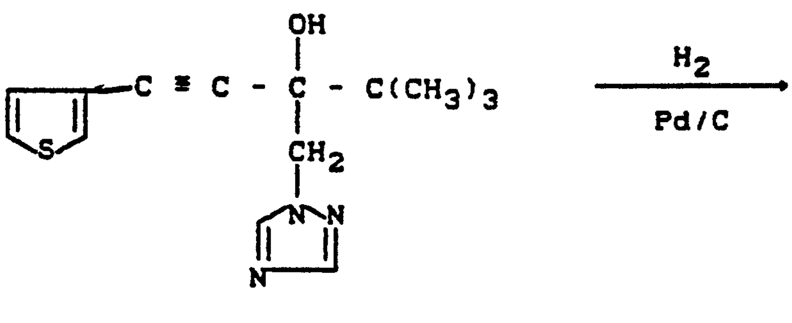


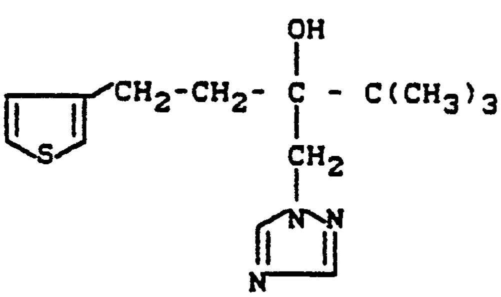

Verwendet man 4,4-Dimethyl-3-hydroxy-1-(3-thienyl)-3-[(1,2,4-triazol-1-yl)-methyl]-1-pentin als Ausgangsstoff und Lithiumaluminium-hydrid als Wasserstoffdonator, so kann der Verlauf des erfindungsgemäßen Verfahrens (b, Variante β) durch das folgende Formelschema wiedergegeben werden:

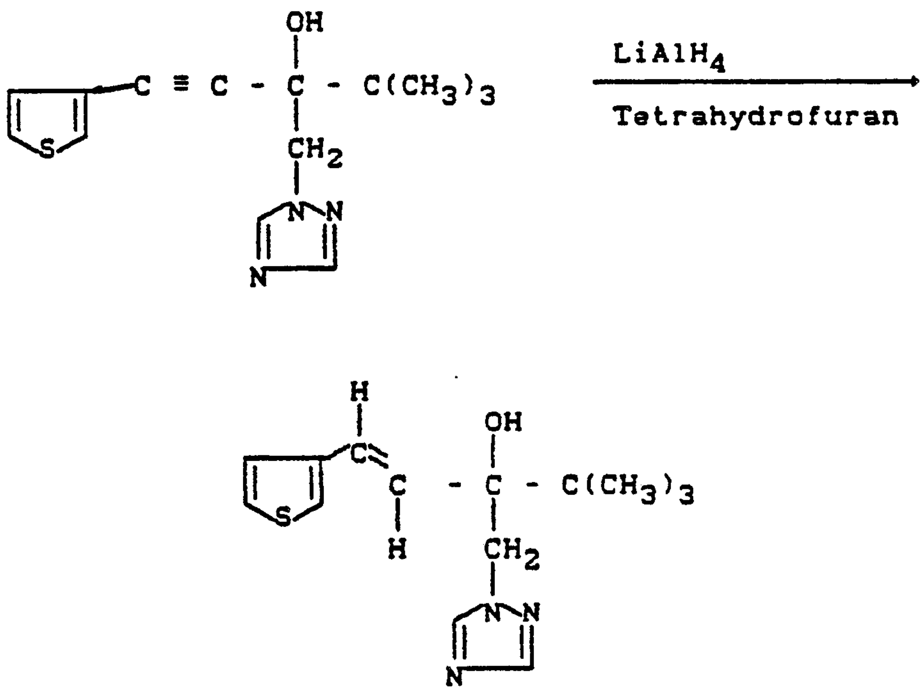


Die bei der Durchführung des erfindungsgemäßen Verfahrens (a) als Ausgangsstoffe benötigten Alkinole sind durch die Formel (II) allgemein definiert. In dieser Formel haben A und $R^1$ vorzugsweise diejenigen Bedeutungen, die bereits im Zusammenhang mit der Beschreibung der erfindungsgemäßen Stoffe der Formel (I) vorzugsweise für diese Reste genannt wurden.

Die Alkinole der Formel (II) sind bisher noch nicht bekannt. Sie lassen sich herstellen, indem man

c) Azolyl-methyl-ketone der Formel

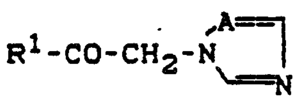 (IV)

in welcher
A und $R^1$ die oben angegebene Bedeutung haben,
mit Acetylen-Salzen der Formel
$HC \equiv CMe$ (V)
in welcher
Me für ein Äquivalent eines Metallkations steht,
in Gegenwart eines Verdünnungsmittels umsetzt,
oder

d) Chlormethylketone der Formel
$R^1 - CO - CH_2Cl$ (VI)
in welcher
$R^1$ die oben angegebene Bedeutung hat, mit Acetylenen der Formel
$HC \equiv CR^2$ (VII)
in welcher
$R^2$ für Wasserstoff oder ein Äquivalent eines Metallkations steht,
gegebenenfalls in Gegenwart einer Base und in Gegenwart eines Verdünnungsmittels umsetzt und dann die dabei entstehenden Hydroxyalkine der Formel

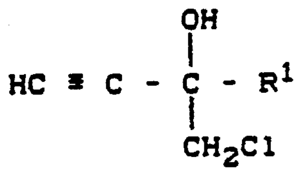

(VIII)

in welcher
$R^1$ die oben angegebene Bedeutung hat,
mit Azolen der Formel

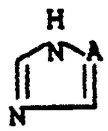

(IX)

in welcher
A die oben angegebene Bedeutung hat,
in Gegenwart eines Säurebindemittels und in Gegenwart eines Verdünnungsmittels umsetzt.

Die bei diesem Verfahren intermediär gebildeten Oxirane der Formel

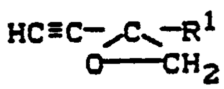

(X)

in welcher
$R^1$ die oben angegebene Bedeutung hat, können auch isoliert und in einem separaten Schritt mit Azolen der Formel (IX) umgesetzt werden.

Die bei der Durchführung des Verfahrens (c) als Ausgangstoffe benötigen Azolyl-methyl-ketone sind durch die Formel (IV) allgemein definiert. In dieser Formel haben A und $R^1$ vorzugsweise diejenigen Bedeutungen, die im Zusammenhang mit der Beschreibung der erfindungsgemäßen Stoffe der Formel (I) bereits vorzugsweise für diese Reste genannt wurden.

Die Azolyl-methyl-ketone der Formel (IV) sind bekannt oder lassen sich nach prinzipiell bekannten Verfahren in einfacher Weise herstellen (vgl. DE-OS 2 431 407).

Die bei dem Verfahren (c) als Reaktionskomponenten benötigten Acetylen-Salze sind durch die Formel (V) allgemein definiert. In dieser Formel steht Me vorzugsweise für ein Lithiumkation oder für ein Äquivalent eines Cer (III)-kations.

Die Acetylen-Salze der Formel (V) sind bekannt (vgl. Houben-Weyl, "Methoden der Organischen Chemie", Bd. V/2a, Seiten 509 ff., Georg Thieme Verlag, Stuttgart 1977 und Tetrahedron Letters **25**, (1984) 4233).

Als Verdünnungsmittel können bei der Durchführung des Verfahrens (c) alle für derartige Umsetzungen üblichen inerten organischen Solventien verwendet werden. Vorzugsweise in Frage kommen Ether, wie Tetrahydrofuran oder Diethylether, und außerdem Kohlenwasserstoffe, wie n-Hexan.

Die Reaktionstemperaturen können bei der Durchführung des Verfahrens (c) innerhalb eines größeren Bereiches variiert werden. Im allgemeinen arbeitet man bei Temperaturen zwischen -78°C und +30°C, vorzugsweise bei Temperaturen zwischen -70°C und +20°C.

Das Verfahren (c) wird ebenso wie die Verfahren (a) und (d) vorzugsweise unter Normaldruck durchgeführt.

Man geht bei der Durchführung des Verfahrens (c) im allgemeinen so vor, daß man zunächst die Acetylen-Salze herstellt und diese dann ohne vorherige Isolierung mit einer äquivalenten Menge, bzw. einem Über- oder Unterschuß an Azolyl-methyl-keton der Formel (IV) umsetzt. Die Aufarbeitung erfolgt nach üblichen Methoden. Im allgemeinen geht man so vor, daß man das Reaktionsgemisch zunächst mit einer wäßrigen Salz-Lösung, zum Beispiel Ammoniumchlorid-Lösung, versetzt, dann mehrfach mit einem in Wasser wenig löslichen organischen Solvens ausschüttelt und die vereinigten organischen Phasen nach dem Trocknen unter vermindertem Druck einengt.

Die bei der Durchführung des Verfahrens (d) als Ausgangsstoffe benötigten Chlormethylketone sind durch die Formel (VI) alalgemein definiert. In dieser Formel hat $R^1$ vorzugsweise diejenigen Bedeutungen,

die bereits im Zusammenhang mit der Beschreibung der erfindungsgemäßen Stoffe der Formel (I) vorzugsweise für diesen Rest genannt wurden.

Die Chlormethylketone der Formel (VI) sind bekannt oder lassen sich nach prinzipiell bekannten Methoden herstellen (vgl. DE-OS 3 049 461).

Die bei dem Verfahren (d) als Reaktionskomponenten benötigten Acetylene sind durch die Formel (VII) allgemein definiert. In dieser Formel steht $R^2$ vorzugsweise für Wasserstoff, ein Lithium-kation oder ein Äquivalent eines Magnesium- oder Cer (III)-kations.

Die Acetylene der Formel (VII) sind bekannt.

Als Basen kommen bei der Durchführung der ersten Stufe des Verfahrens (d) alle für derartige Umsetzungen üblichen starken Basen in Betracht. Vorzugsweise verwendbar sind Alkalimetallhydroxide, wie Kaliumhydroxid.

Als Verdünnungsmittel können bei der Durchführung der ersten Stufe des Verfahrens (d) alle für derartige Umsetzungen üblichen inerten organischen Solventien verwendet werden. Vorzugsweise in Frage kommen Ether, wie Tetrahydrofuran oder Diethylether.

Die Reaktionstemperaturen können bei der Durchführung der ersten Stufe des Verfahrens (d) innerhalb eines größeren Bereichs variiert werden. Im allgemeinen arbeitet man bei Temperaturen zwischen -78° C und +50° C, vorzugsweise zwischen -78° C und +40° C.

Bei der Durchführung der ersten Stufe des Verfahrens (d) geht man im allgemeinen so vor, daß man Chlormethylketone der Formel (VI) und Acetylene der Formel (VII) in angenähert äquivalenten Mengen umsetzt. Es ist jedoch auch möglich, die eine oder andere Komponente in einem Überschuß zu verwenden. Die Aufarbeitung erfolgt nach üblichen Methoden. Die Hydroxyalkine der Formel (VIII) können direkt weiter umgesetzt werden mit Azolen der Formel (IX). Sie können aber auch zunächst in Oxirane der Formel (X) überführt werden und dann mit Azolen der Formel (IX) umgesetzt werden.

Bei der Durchführung der zweiten Stufe des Verfahrens (d) kommen als Säurebindemittel alle üblichen Säureakzeptoren in Frage. Vorzugsweise verwendbar sind Alkalimetallcarbonate und Hydrogencarbonate, wie Natriumcarbonat, Kaliumcarbonat oder Natriumhydrogencarbonat, ferner tertiäre aliphatische oder aromatische Amine, wie Triethylamin, N,N-Dimethyl-cyclohexyl-amin, N,N-Dimethyl-benzylamin und Pyridin, und außerdem cyclische Amine, wie 1,5-Diaza-bicyclo[4.3.0]non-5-en (DBN), 1,8-Diaza-bicyclo[5.4.0]undec-7-en (DBU) und 1,4-Diazabicyclo [2.2.2]octan (DABCO).

Als Verdünnungsmittel kommen bei der Durchführung der zweiten Stufe des Verfahrens (d) alle inerten organischen Solventien in Betracht. Vorzugsweise verwendbar sind aliphatische und aromatische, gegebenenfalls halogenierte Kohlenwasserstoffe wie Pentan, Hexan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Dimethyl- und Dibutylether, sowie tert.-Butylmethylether, Glykoldimethylether und Diglykoldimethylether, Tetrahydrofuran und Dioxan, Ketone wie Aceton, Methylethyl-, Methyl-isopropyl- und Methyl-isobutylketon, Ester wie Essigsäuremethylester und -ethylester, Nitrile wie z.B. Acetonitril und Propionitril, und Pyridin.

Die Reaktionstemperaturen können auch bei der Durchführung der zweiten Stufe des Verfahrens (d) innerhalb eines größeren Bereiches varriert werden. Im allgemeinen arbeitet man bei Temperaturen zwischen 0° C und 200° C, vorzugsweise zwischen 20° C und 150° C.

Bei der Durchführung der zweiten Stufe des Verfahrens (d) geht man im allgemeinen so vor, daß man auf 1 Mol an Hydroxyalkin der Formel (VIII) eine äquivalente Menge oder auch einen Überschuß an Azol der Formel (IX) sowie 2 bis 3 Mol an Säurebindemittel einsetzt. Die Aufarbeitung erfolgt nach üblichen Methoden.

Die bei der Durchführung des erfindungsgemäßen Verfahrens (a) als Reaktionskomponenten benötigten Halogen-Heterocyclen sind durch die Formel (III) allgemein definiert. In dieser Formel steht Het vorzugsweise für diejenigen Bedeutungen, die bereits im Zusammenhang mit der Beschreibung der erfindungsgemäßen Stoffe der Formel (I) vorzugsweise für diesen Rest genannt wurden.

X steht für Chlor, Brom oder Jod, vorzugsweise Brom oder Jod.

Die Halogen-Heterocyclen der Formel (III) sind bekannt oder lassen sich nach prinzipiell bekannten Methoden herstellen.

Als Katalysatoren können bei der Durchführung des erfindungsgemäßen Verfahrens (a) alle für derartige Umsetzungen üblichen Reaktionsbeschleuniger oder Katalysator-Gemische eingesetzt werden. Vorzugsweise verwendbar sind Palladiumkatalysatoren, wie Palladium (II)-acetat, Bis-(triphenylphosphin)-palladium (II)-chlorid, Palladium (II)-chlorid und Tetrakis-(triphenyl-phosphin)-palladium, ferner Gemische aus den zuvor genannten Stoffen unter Zusatz von Phosphinen, wie Triphenylphosphin oder Tri-n-butylphosphin, sowie auch unter Zusatz von Co-Katalysatoren, wie Kupfersalzen, wobei Kupfer (I)-iodid speziell genannt sei.

Als Säurebindemittel kommen bei der Durchführung des erfindungsgemäßen Verfahrens (a) alle

üblichen Säureakzeptoren in Frage. Vorzugsweise verwendbar sind alle diejenigen Säurebindemittel, die bereits im Zusammenhang mit der Beschreibung des Verfahrens (d) vorzugsweise genannt wurden.

Als Verdünnungsmittel kommen bei der Durchführung des erfindungsgemäßen Verfahrens (a) alle üblichen inerten organischen Solventien in Betracht. Vorzugsweise verwendbar sind alle diejenigen Verdünnungsmittel, die bereits im Zusammenhang mit der Beschreibung des Verfahrens (d) vorzugsweise genannt wurden. Außerdem können vorzugsweise auch Amide, wie Dimethylformamid, eingesetzt werden. Verwendet man flüssige Säurebindemittel in einem ausreichenden Überschuß, so erübrigt sich die Zugabe eines zusätzlichen Verdüunnungsmittels.

Die Reaktionstemperaturen können bei der Durchführung des erfindungsgemäßen Verfahrens (a) innerhalb eines größeren Bereiches variiert werden. Im allgemeinen arbeitet man bei Temperaturen zwischen 0°C und 150°C, vorzugseise zwischen 20°C und 100°C.

Bei der Durchführung des erfindungsgemäßen Verfahrens (a) setzt man auf 1 Mol an Alkinol der Formel (II) im allgemeinen 1 bis 4 Mol, vorzugsweise 1 bis 2 Mol an Halogen-Heterocyclen der Formel (III), 0,001 bis 0,1 Mol an Katalysator sowie in bezug auf Alkinol der Formel (II) eine äquivalente Menge oder auch einen Überschuß an Säurebindemittel ein. Die Aufarbeitung erfolgt nach üblichen Methoden. Im allgemeinen geht man so vor, daß man das Reaktionsgemisch einengt, den Rückstand in einem mit Wasser wenig mischbaren organischen Lösungsmittel aufnimmt, dann mehrfach mit Wasser ausschüttelt, anschließend trocknet und einengt.

Die bei der Durchführung des erfindungsgemäßen Verfahrens (b) als Ausgangsstoffe benötigten heterocyclischen Hydroxyethyl-azole sind durch die Formel (Ia) allgemein definiert. Es handelt sich hierbei um erfindungsgemäße Verbindungen, die nach dem Verfahren (a) herstellbar sind.

Bei dem erfindungsgemäßen Verfahren (b, Variante $\alpha$) arbeitet man in flüssiger Phase, in Anwesenheit von Verdünnungsmitteln unter Verwendung eines suspendierten, pulverförmigen Hydrierungskatalysators. Die Durchführung der erfindungsgemäßen Hydrierung kann diskontinuierlich (chargenweise) oder kontinuierlich als Sumpf- oder Rieselphasenhydrierung in bekannten Hydrierreaktoren, wie Autoklaven, Autoklavenkaskaden, Rohrreaktoren oder Umlaufreaktoren erfolgen. Die bevorzugte Arbeitsweise ist die diskontinuierliche Sumpfphasenhydrierung im Autoklaven bei erhöhtem Druck.

Als Verdünnungsmittel kommen bei der Durchführung des erfindungsgemäßen Verfahrens (b, Variante $\alpha$) inerte organische Lösungsmittel in Frage. Hierzu gehören vorzugsweise Alkohole, wie Methanol, Ethanol, Isopropanol oder Ethylenglykol; Ether, wie Diethylether, Diisopropylether, Ethylenglykolmonomethylether, Ethylenglykoldimethylether, Dioxan oder Tetrahydrofuran; gesättigte Kohlenwasserstoffe, wie n-Heptan oder Cyclohexan; sowie Ester, wie Essigsäureethylester.

Für das erfindungsgemäße Verfahren (b, Variante $\alpha$) geeignete Hydrierkatalysatoren sind beispielsweise solche, die aus Metallen und/oder Verbindungen von Elementen der achten Nebengruppe des periodischen Systems der Elemente nach Mendelejew bestehen oder diese enthalten. Dabei sind die Metalle Ruthenium, Rhodium, Palladium, Platin, Kobalt und Nickel und deren Verbindungen bevorzugt. Bei den Metallverbindungen kann es sich beispielsweise um Oxide, Hydroxide und/oder Oxihydrate handeln. Zusätzlich können die Metalle Kupfer, Vanadium, Molybdän, Chrom und/oder Mangan, sowie Verbindungen dieser Metalle zugegen sein.

Die Hydrierkatalysatoren können ausschließlich oder überwiegend aus Wasserstoff-übertragenden Substanzen bestehen, diese können aber auch auf Trägermaterialien aufgebracht sein.

Als Trägermaterialien für die Wasserstoff-übertragenden Substanzen kommen beispielsweise in Frage: anorganische Materialien, wie Kieselgur, Kieselsäure, Aluminiumoxid, Alkali- und Erdalkalisilikate, Aluminiumsilikate, Montmorillonit, Zeolithe, Spinelle, Dolomit, Kaolin, Magnesiumsilikate, Zirkonoxid, Zinkoxid, Calciumcarbonat, Siliciumcarbid, Aluminiumphosphat, Borphosphat, Asbest, Aktivkohle oder Bariumsulfat, aber auch organische Materialien, beispielsweise natürlich vorkommende oder synthetische Verbindungen mit hohen Molekulargewichten wie Seide, Polyamide, Polystyrole, Zellstoff oder Polyurethane. Bevorzugt sind anorganische Trägermaterialien in Pulverform.

Derartige Trägerkatalysatoren können im allgemeinen 0,5 bis 50 Gew-%, vorzugsweise 1 bis 10 Gew.-% der Wasserstoff-übertragenden Substanz, bezogen auf die Gesamtmasse des Trägerkatalysators, enthalten. Die Wasserstoff-übertagende Substanz kann dabei homogen im Trägermaterial verteilt sein, bevorzugt sind jedoch Katalysatoren, in deren äußerer Schicht oder auf deren Oberfläche die Wasserstoff-übertragende Substanz abgelagert ist. Die Herstellung und die Formgebung der Katalysatoren, die im erfindungsgemäßen Verfahren Verwendung finden können, kann in bekannter Weise erfolgen (siehe beispielsweise Houben-Weyl, Methoden der organischen Chemie, Band IV, Ic, Teil I, S. 16 bis 26, Georg Thieme Verlag, Stuttgart, 1980).

Bevorzugte Trägerkatalysatoren sind Ruthenium auf Kohle, Ruthenium auf Aluminiumoxid, Rhodium auf Kohle, Rhodium auf Aluminiumoxid, Palladium auf Kohle, Palladium auf Aluminiumoxid, Palladium auf

Calciumcarbonat, Palladium auf Bariumsulfat, Palladium auf Kieselsäure, Platin auf Kohle und Platin auf Aluminiumoxid, Nickel auf Kieselgur, Nickel auf Alumiumoxid sowie Nickel und Palladium auf Aluminiumoxid.

Bevorzugte Hydrierkatalysatoren, die ausschließlich oder überwiegend aus Wasserstoff-übertragender Substanz bestehen, sind beispielsweise oxidische Katalysatoren, wie Palladiumoxid, Platinoxid, Rutheniumoxid und/oder Rhodiumoxid/Platinoxid nach Nishimura, ferner durch Reduktion von entsprechenden Metallsalzen oder Metallsalzgemischen mit Alkalihydriden, Alkaliboranaten, Metallalkylen, Hydrazin, Formaldehyd, Wasserstoff oder elektropositiven Metallen herstellbare Schwarzkatalysatoren, wie Palladium/Schwarz, Platin/Schwarz und Rhodium/Schwarz; sowie Skelettkatalysatoren vom Raney-Typ, wie Raney-Nickel, Raney-Kobalt, Raney-Nickel-Kobalt, Raney-Nickel-Eisen, Raney-Nickel-Kupfer, Raney-Nickel-Eisen-Chrom, Raney-Nickel-Palladium und Raney-Nickel-Eisen-Vanadium.

Die Hydrierkatalysatoren werden beim erfindungsgemäßen Verfahren (b, Variante α) in einer solchen Menge eingesetzt, daß 0,05 bis 2,5 vorzugsweise 0,1 bis 1 Gew.-% Wasserstoff-übertragende Substanz bezogen auf das Gesamtgewicht des Reaktionsgemisches vorliegen.

Zur Durchführung des erfindungsgemäßen Verfahrens (b, Variante α) können Gemische aus zwei oder mehreren der genannten Hydrierkatalysatoren verwendet werden.

Die katalytische Aktivität der Hydrierkatalysatoren bleibt bei der Durchführung des erfindungsgemäßen Verfahrens im allgemeinen weitgehend erhalten, so daß diese bei diskontinuierlicher Arbeitsweise wiederholt eingesetzt werden können und bei kontinuierlicher Arbeitsweise längere Zeit in Gebrauch bleiben können.

Die Reaktionstemperaturen können in einem größeren Bereich variiert werden. Im allgemeinen arbeitet man im Bereich zwischen 0 °C und 150 °C, vorzugsweise zwischen 20 °C und 120 °C.

Die erfindungsgemäßen Hydrierungen nach Verfahren (b, Variante α) werden vorzugsweise bei erhöhtem Druck durchgeführt. Im allgemeinen arbeitet man zwischen 1 und 150 bar, vorzugsweise zwischen 10 und 30 bar.

Die für das erfindungsgemäße Verfahren (b, Variante α) erforderliche Reaktionszeit ist abhängig von der Reaktionstemperatur, dem Wasserstoffpartialdruck, der Intensität der Durchmischung des Reaktionsgemisches und von der Aktivität und Konzentration des Hydrierkatalysators. Im allgemeinen liegt die erforderliche Reaktionszeit im Bereich von 15 Minuten bis zu mehreren Stunden.

Das erfindungsgemäße Verfahren (b, Variante α) kann beispielsweise in der einfachsten Ausführungsform diskontinuierlich in folgender Weise durchgeführt werden: Ein mit einer Rühr- oder Mischeinrichtung versehener, temperierbarer Autoklav wird in geeigneter Weise mit einem heterocyclischen Hydroxyethylazol der Formel (Ia), dem Hydrierungskatalysator und dem Verdünnungsmittel beschickt. Nachdem der Autoklav entlüftet und sodann Wasserstoff bis zu dem gewünschten Druck aufgedrückt worden ist, wird das Gemisch unter intensiver Durchmischung auf die gewählte Reaktionstemperatur erhitzt. Der Reaktionsverlauf läßt sich leicht durch Messung des Wasserstoffverbrauches, der durch weitere Wasserstoffzufuhr ausgeglichen wird, verfolgen. Die Hydrierung ist beendet, wenn kein Wasserstoff mehr verbraucht wird und die verbrauchte Wasserstoffmenge etwa der theoretisch erforderlichen Wasserstoffmenge entspricht.

Nach beendeter Hydrierung wird das Reaktionsgemisch abgekühlt, entspannt und in bekannter Weise, beispielsweise durch Abfiltrieren des Katalysators und Destillieren des Verdünnungsmittels, aufgearbeitet.

Bei der Durchführung des erfindungsgemäßen Verfahrens (b, Variante β) kommen als Wasserstoffdonatoren vorzugsweise Metalle, wie Zink, Natrium oder Lithium, in Gegenwart von Ammoniak, Ethanol, Ethylamin oder tert.-Butanol sowie von Verdünnungsmittlen, wie Tetrahydrofuran oder 1,2-Dimethoxyethan in Betracht. Auch Chrom(II)-Verbindungen in wäßrigem Medium, wie Chrom(II)-sulfat in Wasser oder Wasser/Dimethylformamid, sind geeignet. Vorzugsweise verwendbar sind ferner Metallhydride, wie Tri-n-butyl-zinnhydrid und Lithiumaluminiumhydrid, Diisobutyl-aluminiumhydrid und Diisobutyl-borhydrid.

Setzt man Metallhydride als Wasserstoffgeneratoren ein, so kommen als Verdünnungsmittel alle für derartige Reaktionen üblichen inerten organischen Solventien in Frage. Vorzugsweise verwendbar sind Ether, wie Tetrahydrofuran, 1,2-Dimethoxyethan und Diethylenglykoldimethylether.

Die Reaktionstemperaturen können bei der Durchführung des erfindungsgemäßen Verfahrens (b, Variante β) innerhalb eines bestimmten Bereiches variiert werden. Im allgemeinen arbeitet man bei Temperaturen zwischen 0 °C und 150 °C, vorzugsweise zwischen 20 °C und 80 °C.

Durch geeignete Wahl von Temperatur, Reaktionszeit und Verdünnungsmittel kann die Reduktion so gelenkt werden, daß entweder Alkene oder Alkane entstehen (vgl. Herstellungsbeispiele).

Bei der Reduktion nach dem Verfahren (b, Variante β) setzt man auf 1 Mol an heterocyclischem Hydroxyethylazol der Formel (Ia) eine solche Menge an Wasserstoffdonator ein, daß die berechnete Menge oder auch ein Überschuß an Wasserstoff erzeugt wird. Die Durchführung der Umsetzung und die Aufarbeitung des Reaktionsgemisches erfolgen nach üblichen Methoden (vgl. Houben-Weyl "Methoden der organischen Chemie", Band V/2a, Seite 687, Georg Thieme Verlag, Stuttgart 1977).

Bei der Durchführung des erfindungsgemäßen Verfahrens (b) kann die Umsetzung sowohl beim Arbeiten nach der Variante $\alpha$ als auch nach der Variante $\beta$ so gelenkt werden, daß entweder diejenigen Verbindungen der Formel (I) entstehen, in denen Y für -CH=CH- steht, oder diejenigen, in denen Y $-CH_2-CH_2-$ bedeutet. Werden Verbindungen hergestellt, in denen Y für -CH=CH- steht, so können diese Stoffe in der E-Form (entgegen) oder in der Z-Form (zusammen) anfallen. Isoliert man Verbindungen der Formel (I), in denen Y für -CH=CH-steht, so können diese Stoffe als Ausgangssubstanzen für die weitere Hydrierung, gegebenenfalls unter anderen Reaktionsbedingungen eingesetzt werden.

Die nach den erfindungsgemäßen Verfahren erhältlichen Verbindungen der Formel (I) können gegebenenfalls noch nach üblichen Methoden halogeniert werden.

Die nach den erfindungsgemäßen Verfahren erhältlichen Verbindungen der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der allgemeinen Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen vorzugsweise diejenigen Salze von Metallen in Frage, die bereits weiter oben beschrieben wurden.

Die Metallsalz-Komplexe von Verbindungen der allgemeinen Formel (I) können in einfacher Weise nach üblichen Verfahren erhalten werden, so z.B. durch Lösen des Metallsalzes in Alkohol, z.B. Ethanol, und Hinzufügen zu Verbindungen der allgemeinen Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;

Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Besonders geeignet sind die erfindungsgemäßen Wirkstoffe zur Bekämpfung von Getreidekrankheiten, wie Leptosphaeria nodorum, Cochliobolus sativus, Pyrenophora teres, Fusarium culmorum und Getreidemehltau, ferner zur Bekämpfung von Gurkenmehltau, Apfelschorf, Bohnenrost und Pyricularia oryzae.

Außerdem besitzen die erfindungsgemäßen Wirkstoffe auch pflanzenwuchsregulierende Eigenschaften.

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden,

daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachs tumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von

Eisen, Mangan, Bor, Kupfer, Kobalt, Molybdän und Zink verwendet werden.

Die Formulierungen enthalten im allgemeinen zwischen 0,1 udn 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und anderen Wachstumsgulatoren.

Die Wirkstoffe können als solche, in Form ihrer Formulierungen oder den deraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von0,0001 bis 0,02 %, am Wirkungsort erforderlich.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg an Wirkstoff.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Die Herstellung und die Verwendung der erfindungsgemäßen Stoffe geht aus den folgenden Beispielen hervor.

## Herstellungsbeispiele

### Beispiel 1

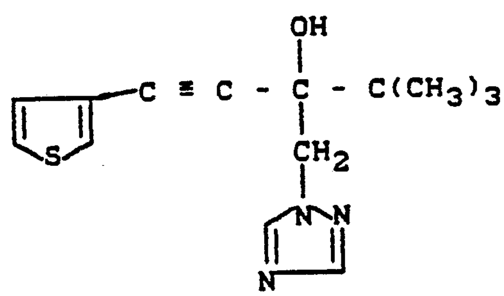

(I-1)

5,8 g (30 mmol) 4,4-Dimethyl-3-hydroxy-3-[(1,2,4-triazolyl-1)-methyl]-1-pentin werden mit 4,9 g (30 mmol) 3-Bromthiophen, 0,2 g Bis-triphenylphosphin-palladiumdichlorid und 25 mg Kupfer (I)-iodid in 60 ml Diethylamin 28 Stunden bei 50 °C geührt. Nach Abziehen von Diethylamin wird das Reaktionsgemisch mit Methylenchlorid verdünnt und mit Wasser mehrfach ausgeschüttelt. Die organische Phase wird über Natriumsulfat getrocknet und unter vermindertem Druck eingeengt. Man erhält 8,1 g (98 % der Theorie) 4,4-Dimethyl-3-hydroxy-1-(3-thienyl)-3[1,2,4-triazolyl-1-)-methyl]-1-pentin vom Schmelzpunkt 148 - 149 °C.

NMR ($CDCl_3$): $\delta$ 1,2 (s., 9H), 3,5 (OH), 4,4 (s, 2H), 6,95 (d, 1H), 7,25 (d, 1H), 7,3 (s, 1H), 8,0 (s, 1H), 8,3 (s, 1H).

Herstellung des Vorprodukts

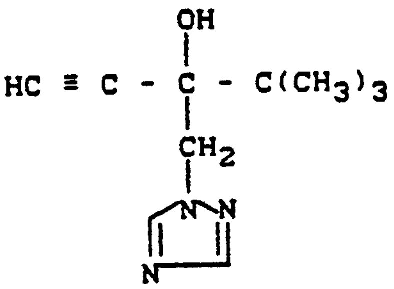

(II-1)

In 880 ml absolutem Tetrahydrofuran werden bei 70° C 13 g (0,5 mol) Acetylen eingeleitet und durch Zutropfen von 200 ml (0,5 mol) Butyllithium in Hexan metalliert. Nach 30 Minuten wird eine Lösung von 78,5 g (0,47 mol) 3,3-Dimethyl-1-(1,2,4-triazolyl-1)-2-butanon in 150 ml absolutem Tetrahydrofuran bei -70° C zugetropft. Das Reaktionsgemisch wird 2 Stunden bei -70° C nachgerührt, dann aufgetaut und noch 2 Stunden bei 20° C gerührt. Nach dem Verdünnen mit gesättigter, wäßriger Ammonium -chlorid-Lösung wird mehrfach mit Methylenchlorid ausgeschüttelt. Die vereinigten organischen Phasen werden getrocknet und dann unter vermindertem Druck eingeengt. Es verbleiben 74 g eines Produktes, das nach gaschromatographischer Analyse zu 38 % aus 3,3-Dimethyl-1-(1,2,4-triazol-1-yl)-2-butanon und zu 57 % aus 4,4-Dimethyl-3-hydroxy-3-[(1,2,4-triazol-1-yl)-methyl]-1-pentin besteht. Nach Umkristallisation aus Toluol erhält man reines 4,4-Dimethyl-3-hydroxy-[(1,2,4-triazol-1-yl)-methyl]-1-pentin vom Schmelzpunkt 129 - 131° C.
NMR ($CDCl_3$): δ 1,2 (s, 9H), 2,35 (s, 1H), 3,75 (OH), 4,4 (AB, 2H) , 8,0 (s, 1H), 8,25 (s, 1H).

Beispiel 2

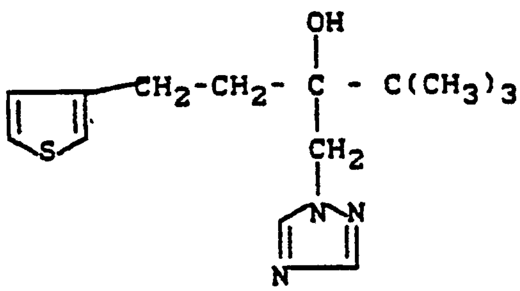

(I-2)

2,3 g (8,4 mmol) 4,4-Dimethyl-3-hydroxy-1-(3-thienyl)-3-[(1,2,4-triazolyl-1)-methyl]-1-pentin werden in 50 ml Essigsäureethylester über 1 g 5 % Palladium auf Aktivkohle mit 20 bar Wasserstoff bei 120° C 12 Stunden hydriert. Nach Filtration des Reaktionsgmeisches und Abziehen des Lösungsmittels bleiben 2,2 g (95 % der Theorie) 4,4-Dimethyl-3-hydroxy-1-(3-thienyl)-3-[(1,2,4-triazolyl-1-)-methyl]-pentan zurück, das langsam kristallisiert. Schmelzpunkt: 84 - 88° C.
NMR ($CDCl_3$): δ 1,05 (s, 9H), 1,7 - 2,7 (m, 4H), 4,35 (s, 2H), 4,8 (OH), 6,85 (d, 1H), 6,9 (s, 1H) 7,1 (d, 1H), 8,0 (s, 1H), 8,3 (s, 1H).

Beispiel 3

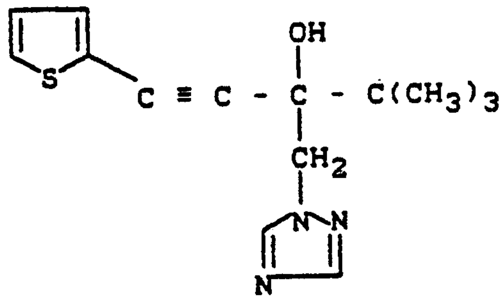

(I-3)

19,3 g (0,1 mol) 4,4-Dimethyl-3-hydroxy-3-[(1,2,4-triazolyl-1)-methyl]-1-pentin und 16,3 g (0,1 mol) 2-Bromthiophen werden in 300 ml Triethylamin mit 1 g Bistriphenylphosphinpalladiumdichlorid und 0,125 g Kupfer (I)-iodid 23 h bei 50 °C gerührt. Nach Aufarbeitung mit Methylenchlorid und Wasser erhält man 22,5 g (82 % der Theorie) 4,4-Dimethyl-3-hydroxy-1-(2-thienyl)-3-[(1,2,4-triazolyl-1)-methyl]-1-pentin vom Schmelzpunkt 125 - 128 °C.
NMR ($CDCl_3$): δ 1,2 (s, 9H), 4,15 (OH), 4,45 (AB, 2H) 6,9 (2 d, 1H) 7,05 (d, 1H), 7,2 (d, 1H), 8,0 (s, 1H), 8,25 (s, 1H).

Beispiel 4

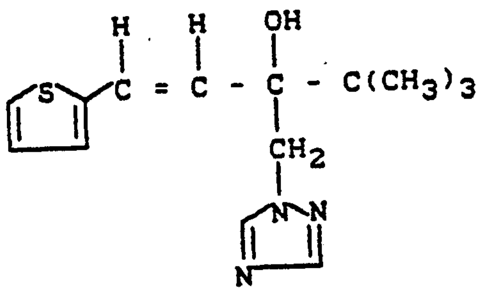

(I-4)

2,75 g (10 mmol) 4,4-Dimethyl-3-hydroxy-1-(2-thienyl)-3-[(1,2,4-triazolyl-1)-methyl]-1-pentin werden in 50 ml Essigsäureethylester über 1 g 5 % Palladium auf Aktivkohle bei 80 °C mit 10 bar Wasserstoff hydriert. Nach Filtration des Reaktionsgemisches und Abziehen des Lösungsmittels erhält man 2,1 g (76 % der Theorie) 4,4-Dimethyl-3-hydroxy-1-(2-thienyl)-3-[(1,2,4-triazolyl-1)-methyl]-1(Z)-penten.
NMR ($CDCl_3$): δ 1,1 (s, 9H), 4,3 (OH), 4,4 (AB, 2H) 5,5 (d, J = 13 Hz, 1H), 6,5 (d, J = 13 Hz, 1H), 6,8 - 7,3 (m, 3H), 7,9 (s, 1H), 8,25 (s, 1H).

Beispiel 5

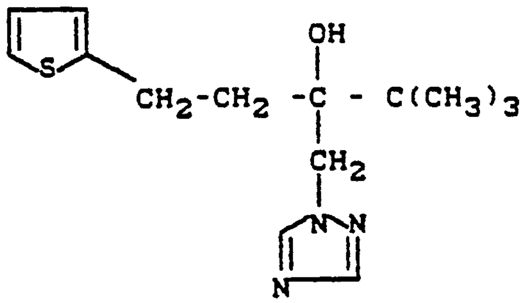

(I-5)

2,8 g (10 mmol) 4,4-Dimethyl-3-hydroxy-1-(2-thienyl)-3-[(1,2,4-triazolyl-1-)methyl]-1(Z)-penten werden in 50 ml Methanol über 1 g 5 % Palladium auf Aktivkohle bei 100 °C mit 20 bar Wasserstoff 12 Stunden hydriert. Nach Filtration des Reaktionsgemisches und Abziehen des Lösungsmittels bleiben 2,6 g (93 % der Theorie) 4,4-Dimethyl-3-hydroxy-1-(2-thienyl)-3-[(1,2,4-triazolyl-1)-methyl]-pentan zurück.

NMR ($CDCl_3$): δ 1,0 (s, 9H), 1,8 - 2,2 (m, 4H), 3,5 (OH), 4,35 (s, 2H), 6,65 (d, 1H), 6.85 (m, 1H) 7,05 (d, 1H), 8,0 (s, 1H), 8,25 (s, 1H).

Die Verbindung (I-5) wird auch erhalten, wenn man zu 0,76 g (20 mmol) Lithiumaluminium-hydrid in 20 ml absolutem Diethylenglykoldimethylether 2,75 g (10 mmol) 4,4-Dimethyl-3-hydroxy-1-(2-thienyl)-3-[(1,2,4-triazol-1-yl)-methyl]-1-pentin gibt und 17 Stunden auf 120 °C erhitzt. Nach dem Abkühlen auf Raumtemperatur werden 5 ml konzentrierte wäßrige Ammoniaklösung zugetropft. Das Reaktionsgemisch wird 10 Minuten gerührt und dann mit 100 ml Diethylether verdünnt. Man filtriert über Celite, trocknet über Natriumsulfat und engt unter vermindertem Druck ein. Es verbleiben 2,3 g (82 % der Theorie) an 4,4-Dimethyl-3-hydroxy-1-(2-thienyl)-3-[(1,2,4-triazol-1-yl)-methyl]-pentan.

Beispiel 6

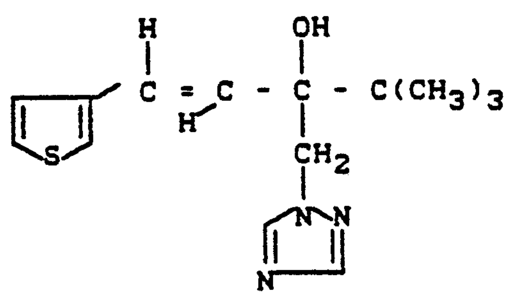

(I-6)

Zu einem Gemisch aus 0,76 g (20 mmol) Lithiumaluminiumhydrid in 15 ml absoutem Tetrahydrofuran wird bei Raumtemperatur unter Rühren eine Lösung von 2,75 g (10 mmol) 4,4-Dimethyl-3-hydroxy-1-(3-thienyl)-3-[(1,2,4-triazol-1-yl)-methyl]-1-pentin in 10 ml absolutem Tetrahydrofuran getropft. Es wird 4 Stunden unter Rückfluß erhitzt. Danach wird des Reaktionsgemisch auf 0 bis 5 °C abgekühlt und tropfenweise mit 5 ml konzentriertem Ammoniak versetzt. Man verdünnt mit Diethylether, filtriert über Celite, trocknet und engt unter vermindertem Druck ein. Es verbleiben 1,9 g (69 % der Theorie) an 4,4-Dimethyl-3-hydroxy-1-(3-thienyl)-3-[(1,2,4-triazol-1-yl)-methyl]-1(E)-penten vom Schmelzpunkt 96 °C.

NMR ($CDCl_3$): δ 1,1 (s, 9H), 3,6 (OH), 4,4 (AB, 2H), 6,05 (d, J = 15 Hz, 1H), 6,4 (d, J = 15 Hz, 1H), 7,05 (m, 2H), 7,2 (m, 1H), 7,85 (s, 1H), 8,05 (s, 1H).

In entsprechender Weise gemäß den angegebenen Verfahrensbedingungen werden die in der folgenden Tabelle formelmäßig aufgeführten Verbindungen der Formel

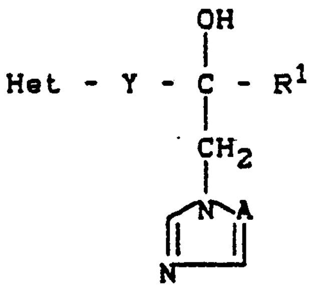

(I)

erhalten.

Tabelle 2

| Bsp. Nr. | $R^1$ | Y | A | Het | Schmelzpunkt [°C] |
|---|---|---|---|---|---|
| 7 | $(CH_3)_3C$ | -C≡C- | N | 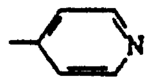 | 117 - 118 |
| 8 | $(CH_3)_3C$ | -C≡C- | N | 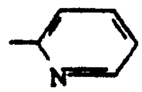 | 136 - 140 |
| 9 | $(CH_3)_3C$ | -C≡C- | N | 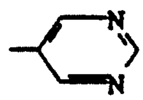 | 147 - 150 |
| 10 | $(CH_3)_3C$ | -C≡C- | N | 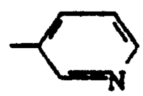 | 118 - 121 |
| 11 | $(CH_3)_3C$ | -C≡C- | N | 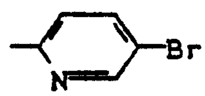 | 182 - 184 |
| 12 | $(CH_3)_3C$ | -C≡C- | N | 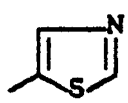 | 164 - 168 |
| 13 | $(CH_3)_3C$ | -C≡C- | N | 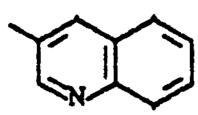 | 154 - 156 |
| 14 | $(CH_3)_3C$ | $-CH_2-CH_2-$ | N | 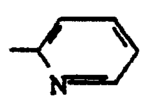 | Öl |

Tabelle 2 (Fortsetzung)

| Bsp. Nr. | $R^1$ | Y | A | Het | Schmelzpunkt [° C] |
|---|---|---|---|---|---|
| 15 | $(CH_3)_3C$ | -C≡C- | N | 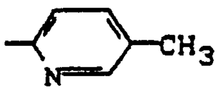 | 179 - 182 |
| 16 | $(CH_3)_3C$ | $-CH_2-CH_2-$ | N | 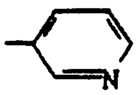 | 102 - 107 |

**Tabelle 2** (Fortsetzung)

| Bsp. Nr. | $R^1$ | Y | A | Het | Schmelzpunkt [°C] |
|---|---|---|---|---|---|
| 17 | $(CH_3)_3C$ | $-CH_2-CH_2-$ | N | 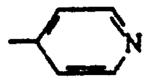 | 115 |
| 18 | $(CH_3)_3C$ | " | N | 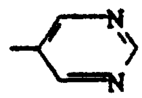 | Öl |
| 19 | $(CH_3)_3C$ | $-CH=CH-$ (Z) | N | 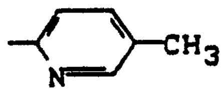 | 89 - 93 |
| 20 | $(CH_3)_3C$ | $-C\equiv C-$ | N | 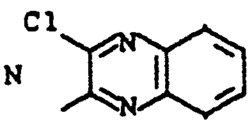 | 173 - 177 |
| 21 | $(CH_3)_3C$ | $-CH_2-CH_2$ | N | 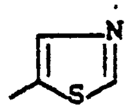 | 100 - 104 |
| 22 | $(CH_3)_3C$ | $-C\equiv C-$ | N | 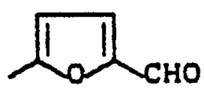 | 112 - 115 |
| 23 | $(CH_3)_3C$ | $-C\equiv C-$ | N | 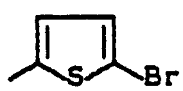 | 94 - 98 |
| 24 | $(CH_3)_3C$ | $-C\equiv C-$ | | 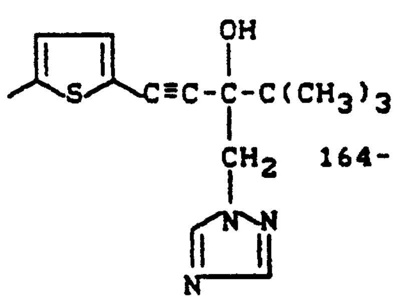 | 164-166 |

Tabelle 2 (Fortsetzung)

| Bsp. Nr. | $R^1$ | Y | A | Het | Schmelzpunkt [°C] |
|---|---|---|---|---|---|
| 25 | $(CH_3)_3C$ | $-C\equiv C-$ | N | 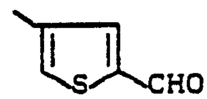 | 136 - 140 |
| 26 | $(CH_3)_3C$ | $-C\equiv C-$ | N | 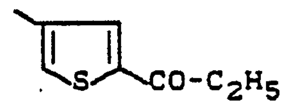 | 80 - 82 |
| 27 | $(CH_3)_3C$ | $-C\equiv C-$ | N | 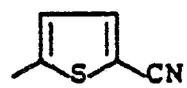 | 101 - 106 |
| 28 | $(CH_3)_3C$ | $-C\equiv C-$ | N | 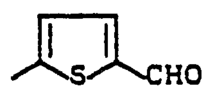 | 162 |
| 29 | $(CH_3)_3C$ | $-CH_2-CH_2-$ | N | 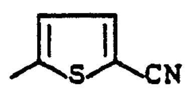 | Öl |
| 30 | $(CH_3)_3C$ | $-CH_2-CH_2-$ | N | 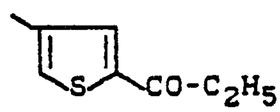 | 108 |
| 31 | $(CH_3)_3C$ | $-C\equiv C-$ | N | 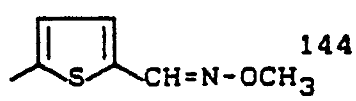 | 144 - 148 |

Tabelle 2 (Fortsetzung)

| Bsp. Nr. | $R^1$ | Y | A | Het | Schmelzpunkt [°C] |
|---|---|---|---|---|---|
| 32 | $(CH_3)_3C$ | -C≡C- | N | 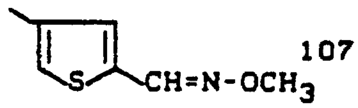 | 107 - 112 |
| 33 | $(CH_3)_3C$ | -C≡C- | N | 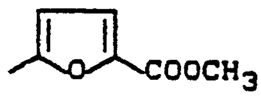 | 141 - 143 |
| 34 | $(CH_3)_3C$ | -C≡C- | N | 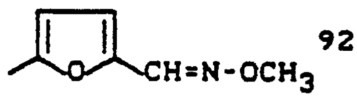 | 92 - 96 |
| 35 | $(CH_3)_3C$ | $-CH_2-CH_2-$ | N | 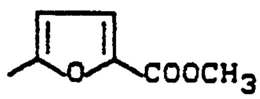 | 118 - 123 |
| 36 | $(CH_3)_3C$ | (E)-CH=CH- | N | 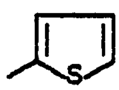 | 94 - 97 |
| 37 | $(CH_3)_3C$ | -C≡C- | N | 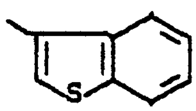 | 102 - 104 |
| 38 | $(CH_3)_3C$ | -C≡C- | N | 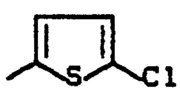 | 105 |
| 39 | $(CH_3)_3C$ | $-CH_2-CH_2-$ | N | 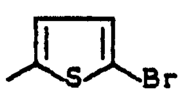 | Öl |
| 40 | $(CH_3)_3C$ | $-CH_2-CH_2-$ | N | 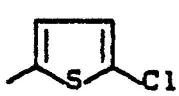 | Öl |

<u>Tabelle 2</u> (Fortsetzung)

| Bsp. Nr. | $R^1$ | Y | A | Het | Schmelzpunkt [°C] |
|---|---|---|---|---|---|
| 41 | $(CH_3)_3C$ | $-CH_2-CH_2-$ | N | 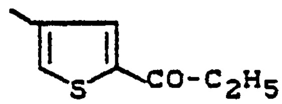 | 108 |
| 42 | $(CH_3)_3C$ | $-CH_2-CH_2-$ | N | 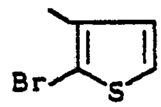 | 118-121 |
| 43 | $(CH_3)_3C$ | $-CH_2-CH_2-$ | N | 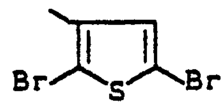 | 138-140 |
| 44 | $(CH_3)_3C$ | $-C\equiv C-$ | N | 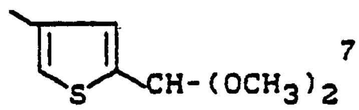 | 79-83 |
| 45 | $(CH_3)_3C$ | $-C\equiv C-$ | N | 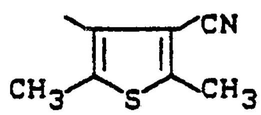 | 75 |
| 46 | 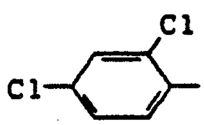 | $-C\equiv C$ | N | 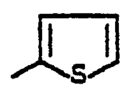 | 145 - 148 |
| 47 | 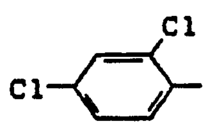 | $-C\equiv C-$ | N | 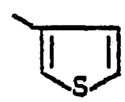 | Öl |

Tabelle 2 (Fortsetzung)

| Bsp. Nr. | $R^1$ | Y | A | Het | Schmelzpunkt [°C] |
|---|---|---|---|---|---|
| 48 | 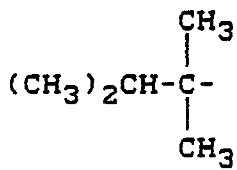 | -C≡C- | N | 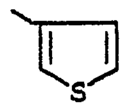 | Öl |
| 49 | 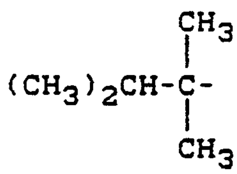 | -C≡C- | N | 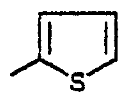 | 88-91 |
| 50 | 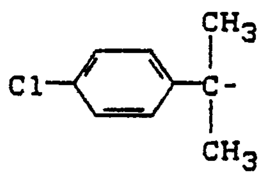 | $-CH_2-CH_2-$ | N | 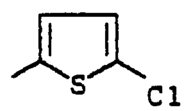 | 106-108 |
| 51 | 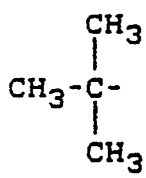 | $-CH_2-CH_2-$ | N | 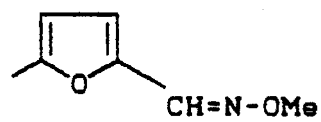 | Öl |
| 52 | 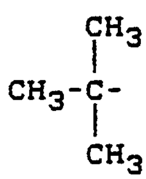 | $-CH_2-CH_2-$ | N | 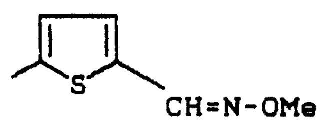 | 95-97 |
| 53 | 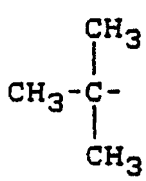 | $-CH_2-CH_2-$ | N | 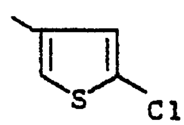 | 134-136 |

Tabelle 2 (Fortsetzung)

| Bsp. Nr. | $R^1$ | Y | A | Het | Schmelzpunkt [°C] |
|---|---|---|---|---|---|
| 54 | 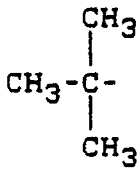 | $-CH_2-CH_2-$ | N | 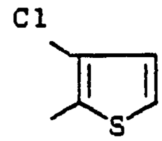 | 90 |
| 55 | 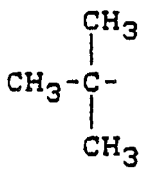 | $-CH_2-CH_2-$ | N | 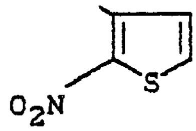 | Öl |
| 56 | 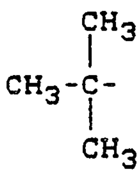 | $-C\equiv C-$ | N | 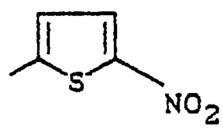 | 138-143 |
| 57 | 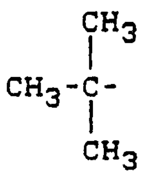 | $-C\equiv C-$ | N | 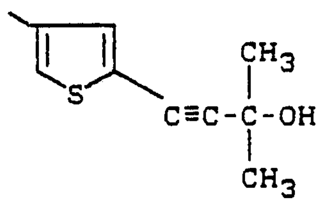 | 149-153 |
| 58 | 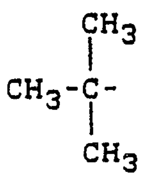 | $-CH_2-CH_2-$ | N | 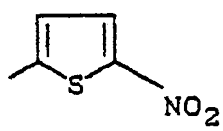 | Öl |
| 59 | 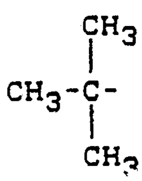 | $-C\equiv C-$ | N | 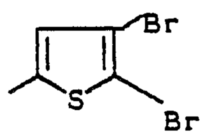 | 141-143 |

<u>Tabelle 2</u> (Fortsetzung)

| Bsp. Nr. | $R^1$ | Y | A | Het | Schmelzpunkt [°C] |
|---|---|---|---|---|---|
| 60 | 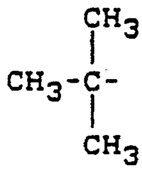 | $-CH_2-CH_2-$ | N | 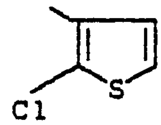 | 94 |
| 61 | 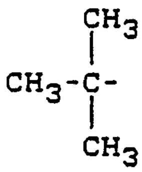 | -C≡C- | N | 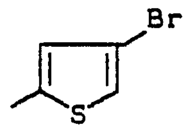 | 117-120 |
| 62 | 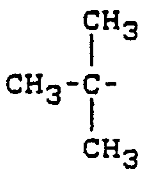 | -C≡C- | N | 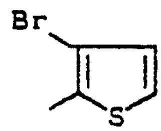 | Öl |
| 63 | 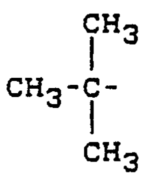 | -C≡C- | | 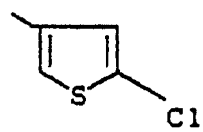 | 94-95 |
| 64 | 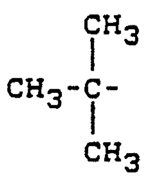 | -C≡C- | N | 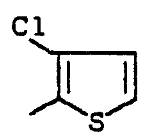 | 103-105 |
| 65 | 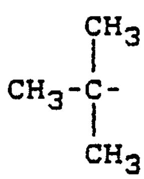 | -C≡C- | N | 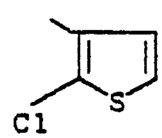 | 140-142 |

Tabelle 2 (Fortsetzung)

| Bsp. Nr. | $R^1$ | Y | A | Het | Schmelzpunkt [°C] |
|---|---|---|---|---|---|
| 66 | 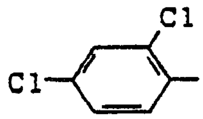 | $-CH_2-CH_2-$ | N | 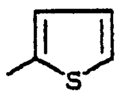 | 98-101 |
| 67 | 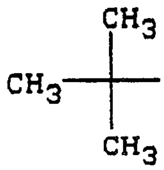 | $-CH=CH-$ E | N | 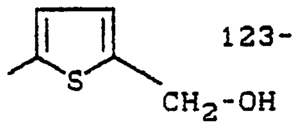 | 123-125 |
| 68 | 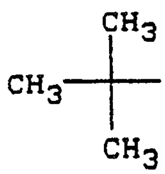 | $-CH=CH-$ E | N | 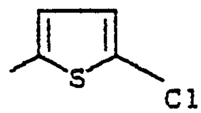 | Öl |
| 69 | 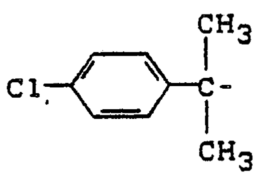 | $-C{\equiv}C-$ | N | 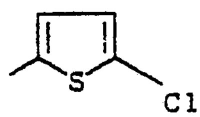 | Öl |
| 70 | 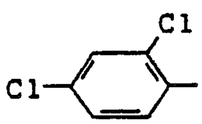 | $-C{\equiv}C-$ | N | 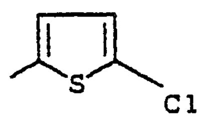 | 64 |
| 71 | 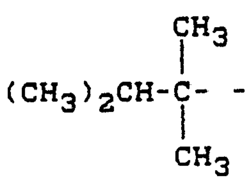 | $-CH_2-CH_2$ | N | 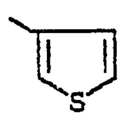 | Öl |
| 72 | 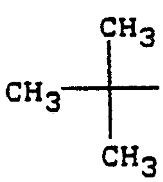 | $-CH_2-CH_2-$ | N | 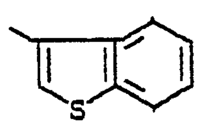 | Öl |

In den folgenden Verwendungsbeispielen wurden die nachstehend angegebenen Verbindungen als Vergleichssubstanzen eingesetzt:

(A) = 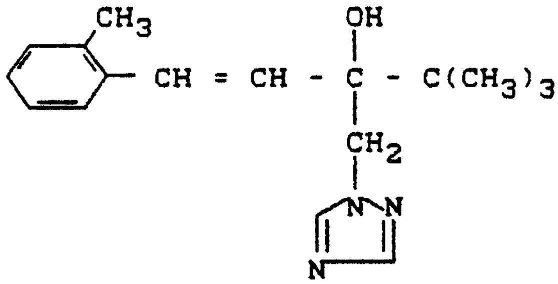

(B) = 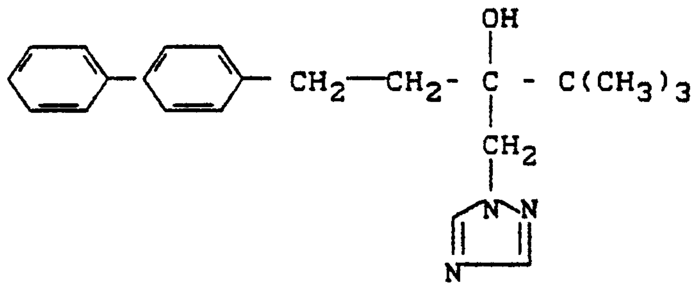

(C) = 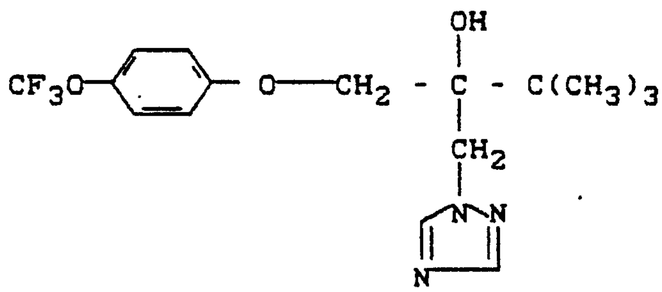

(D) = 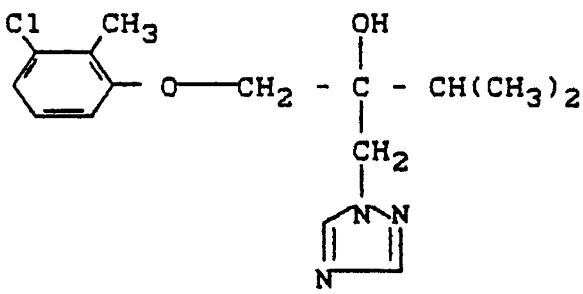

Beispiel A

Leptosphaeria nodorum (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 15 °C und einer relativen Luftfeuchtigkeit von ca. 80 % aufgestellt.

10 Tage nach der Inokulation erfolgt die Auswertung.

In diesem Test zeigen die in den Beispielen 1, 2, 10 und 31 aufgeführten erfindungsgemäßen Stoffe eine wesentlich bessere Wirksamkeit als die Vergleichssubstanzen (A), (B), (C) und (D).

## Ansprüche

1. Heterocyclische Hydroxyethyl-azole der Formel

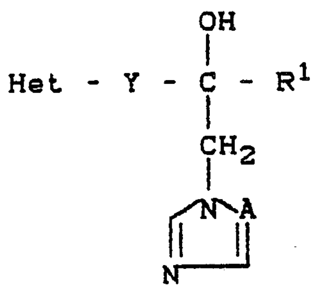

(I)

in welcher
A für ein Stickstoffatom oder eine CH-Gruppe steht,
Y für die Gruppierungen $-CH_2-CH_2-$, $-CH=CH-$ oder $-C\equiv C-$steht,
$R^1$ für gegebenenfalls substituiertes Alkyl, gegebenenfalls substituiertes Alkenyl, gegebenenfalls substituiertes Cycloalkyl oder für gegebenenfalls substituiertes Aryl steht und
Het für einen gegebenenfalls substituierten und gegebenenfalls benzanellierten, fünf- oder sechsgliedrigen heterocyclischen Rest mit bis zu 3 Heteroatomen steht,
sowie deren Säureadditions-Salze und Metallsalz-Komplexe.

2. Heterocyclische Hydroxyethyl-azole der Formel (I) gemäß Anspruch 1, in denen
A für ein Stickstoffatom oder eine CH-Gruppe steht,
Y für die Gruppierungen $-CH_2-CH_2$, $-CH=CH-$ oder $-C\equiv C-$ steht,
$R^1$ für geradkettiges oder verzweigtes Alkyl mit 3 bis 7 Kohlenstoffatomen steht, wobei jeder dieser Reste substituiert sein kann durch Halogen, Cycloalkyl mit 3 bis 7 Kohlenstoffatomen, Phenyl und/oder Halogenphenyl,
für gegebenenfalls durch Halogen, Phenyl und/oder Halogenphenyl substituiertes Alkenyl mit 3 bis 6 Kohlenstoffatomen steht, ferner für Cycloalkyl mit 3 bis 7 Kohlenstoffatomen steht, wobei jeder dieser Reste durch Alkyl mit 1 bis 4 Kohlenstoffatomen substituiert sein kann, oder für Phenyl steht, das einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschieden Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
und
Het für einen gegebenenfalls benzanellierten fünf gliedrigen Heterocyclus mit 1 bis 3 Stickstoffatomen, für einen gegebenenfalls benzanellierten fünfgliedrigen Heterocyclus mit einem Sauerstoffatom, für einen gegebenenfalls benzanellierten fünfgliedrigen Heterocyclus mit einem Schwefelatom, für einen gegebenenfalls benzanellierten fünfgliedrigen Heterocyclus mit einem Sauerstoffatom und einem Schwefelatom, für einen gegebenenfalls benzanellierten fünfgliedrigen Heterocyclus mit einem Sauerstoffatom und einem Stickstoffatom, für einen gegebenenfalls benzanellierten fünfgliedrigen Heterocyclus mit einem Schwefelatom und einem Stickstoffatom oder für einen gegebenenfalls benzanellierten sechsgliedrigen Heterocyclus

mit 1 bis 3 Stickstoffatomen steht, wobei jeder der zuvor ge nannten heterocyclischen Reste ein- oder mehrfach, gleichartig oder verschieden substituiert sein kann durch Halogen, Alkyl mit 1 bis 4 Kohlenstoffatomen, Hydroxyalkyl mit 1 bis 4 Kohlenstoffatomen, Hydroxyalkinyl mit 3 bis 8 Kohlenstoffatomen, Alkoxy mit 1 oder 2 Kohlenstoffatomen, Alkylthio mit 1 oder 2 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, Formyl, Dialkoxymethyl mit 1 oder 2 Kohlenstoffatomen in jedem Alkoxyteil, Acyl mit 2 bis 4 Kohlenstoffatomen, Alkoxycarbonyl mit 1 bis 4 Kohlenstoffatomen im Alkoxylteil, Alkoximinoalkyl mit 1 bis 4 Kohlenstoffatomen im Alkoxyteil und 1 bis 3 Kohlenstoffatomen im Alkylteil, Nitro und/oder Cyano oder durch den Rest der Formel

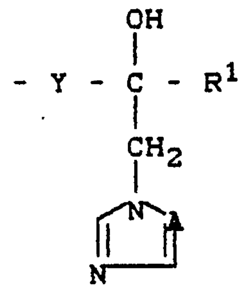
,

wobei $R^1$, A und Y die oben angegebene Bedeutung haben.

3. Verfahren zur Herstellung von heterocyclischen Hydroxyethyl-azolen der Formel

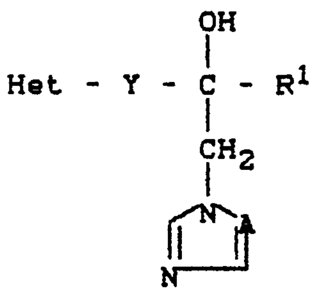
(I)

in welcher
A für ein Stickstoffatom oder eine CH-Gruppe steht,
Y für die Gruppierungen $-CH_2-CH_2-$, $-CH=CH-$ oder $-C\equiv C-$steht,
$R^1$ für gegebenenfalls substituiertes Alkyl, gegebenenfalls substituiertes Alkenyl, gegebenenfalls substituiertes Cycloalkyl oder für gegebenenfalls substituiertes Aryl steht und
Het für einen gegebenenfalls substituierten und gegebenenfalls benzanellierten, fünf- oder sechsgliedrigen heterocyclischen Rest mit bis zu 3 Heteroatomen steht,
sowie von deren Säureadditions-Salzen und Metallsalz-Komplexen, dadurch gekennzeichnet, daß man
a) Alkinole der Formel

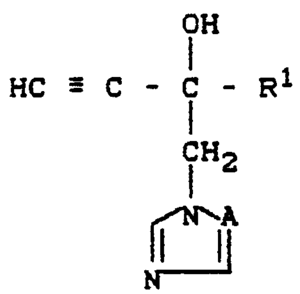
(II)

in welcher
A und $R^1$ die oben angebebene Bedeutung haben,
mit Halogen-Heterocyclen der Formel
Het - X (III)

in welcher
Het die oben angegebene Bedeutung hat und
X für Chlor, Brom oder Jod steht,
in Gegenwart eines Säurebindemittels und in Gegenwart eines Katalysators sowie in Gegenwart eines Verdünnungsmittels umsetzt,
oder
b) heterocyclische Hydroxylethyl-azole der Formel

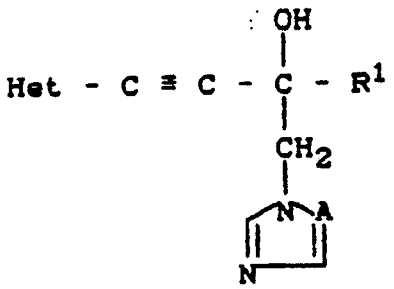
(Ia)

in welcher
Het, A und $R^1$ die oben angegebene Bedeutung haben,
entweder
α) mit Wasserstoff in Gegenwart eines Hydrierkatalysators und in Gegenwart eines Verdünnungsmittels umsetzt,
oder
β) mit Wasserstoffdonatoren in Gegenwart eines Verdünnungsmittels umsetzt,
und gegebenenfalls halogeniert und gegebenenfalls anschließend an die so erhaltenen Verbindungen der Formel (I) eine Säure oder ein Metallsalz addiert.

4. Fungizide und pflanzenwuchsregulierende Mittel, gekennzeichnet durch einen Gehalt an mindestens einem heterocyclischen Hydroxyethyl-azol der Formel (I) gemäß Anspruch 1 bzw. an einem Säureadditions-Salz oder Metallsalz-Komplex eines heterocyclischen Hydroxyethyl-azols der Formel (I).

5. Verwendung von heterocyclischen Hydroxyethyl-azolen der Formel (I) gemäß Anspruch 1 bzw. von deren Säureadditions-Salzen und Metallsalz-Komplexen zur Bekämpfung von Pilzen sowie zur Regulierung des Pflanzenwachstums.

6. Verfahren zur Bekämpfung von Pilzen und zur Regulierung des Pflanzenwachstums, dadurch gekennzeichnet, daß man heterocyclische Hydroxyethylazole der Formel (I) gemäß Anspruch 1 bzw. deren Säureadditions-Salze oder Metallsalz-Komplexe auf die Pilze bzw. Pflanzen oder deren Lebensraum ausbringt.

7. Verfahren zur Herstellung von fungiziden und pflanzenwuchsregulierenden Mitteln, dadurch gekennzeichnet, daß man heterocyclische Hydroxyethyl-azole der Formel (I) gemäß Anspruch 1 bzw. deren Säureadditionssalze oder Metallsalz-Komplexe mit Streckmitteln und/oder oberflächenaktiven Stoffen vermischt.

8. Alkinole der Formel

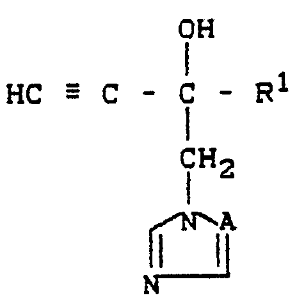
(II)

in welcher
A für ein Stickstoffatom oder eine CH-Gruppe steht und
$R^1$ für gegebenenfalls substituiertes Alkyl, gegebenenfalls substituiertes Alkenyl, gegebenenfalls substituiertes Cycloalkyl oder für gegebenenfalls substituiertes Aryl steht.

9. Verfahren zur Herstellung von Alkinolen der Formel

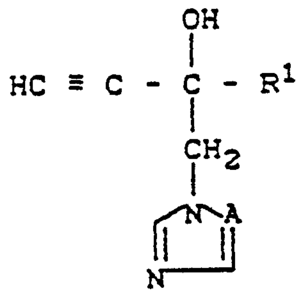

(II)

in welcher
A für ein Stickstoffatom oder eine CH-Gruppe steht und
$R^1$ für gegebenenfalls substituiertes Alkyl, gegebenenfalls substituiertes Alkenyl, gegebenenfalls substituiertes Cycloalkyl oder für gegebenenfalls substituiertes Aryl steht,
dadurch gekennzeichnet, daß man
c) Azolyl-methyl-ketone der Formel

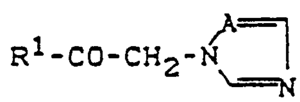

(IV)

in welcher
A und $R^1$ die oben angegebene Bedeutung haben,
mit Acetylen-Salzen der Formel
$HC \equiv CMe$ (V)
in welcher
Me für ein Äquivalent eines Metallkations steht,
in Gegenwart eines Verdünnungsmittels umsetzt,
oder
d) Chlormethylketone der Formel
$R^1 - CO - CH_2Cl$ (VI)
in welcher
$R^1$ die oben angegebene Bedeutung hat, mit Acetylenen der Formel
$HC \equiv CR^2$ (VII)
in welcher
$R^2$ für Wasserstoff oder ein Äquivalent eines Metallkations steht,
gegebenenfalls in Gegenwart einer Base und in Gegenwart eines Verdünnungsmittels umsetzt und dann die dabei entstehenden Hydroxyalkine der Formel

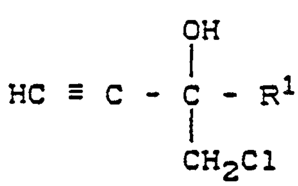

(VIII)

in welcher
$R^1$ die oben angegebene Bedeutung hat,
mit Azolen der Formel

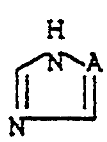

(IX)

in welcher
A die oben angegebene Bedeutung hat,
in Gegenwart eines Säurebindemittels und in Gegenwart eines Verdünnungsmittels umsetzt.

Europäisches Patentamt

# EUROPÄISCHER RECHERCHENBERICHT

Nummer der Anmeldung

EP 88 10 8238

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 097 426 (I.C.I.)<br>* Seite 33 * | 8 | C 07 D 409/06<br>C 07 D 405/06<br>C 07 D 403/06<br>C 07 D 401/06<br>C 07 D 417/06<br>C 07 D 249/08<br>C 07 D 233/60<br>A 01 N 43/653<br>A 01 N 43/50 |
| Y | EP-A-0 052 424 (I.C.I.)<br>* Seiten 4,9 * | 1-7 | |
| Y | EP-A-0 195 557 (PFIZER CO.)<br>* Seite 2 * | 1-7 | |
| Y | EP-A-0 193 642 (BAYER AG)<br>* Seiten 2,3 * | 1-7 | |
| D,Y | EP-A-0 207 590 (I.C.I.)<br>* Ansprüche * | 1-7 | |
| D,Y | EP-A-0 212 841 (I.C.I.)<br>* Ansprüche * | 1-7 | |

RECHERCHIERTE SACHGEBIETE (Int. Cl.4)

C 07 D 409/00
C 07 D 405/00
C 07 D 403/00
C 07 D 401/00
C 07 D 417/00
C 07 D 249/00
C 07 D 233/00
A 01 N 43/00

Der vorliegende Recherchenbericht wurde für alle Patentansprüche erstellt

| Recherchenort | Abschlußdatum der Recherche | Prüfer |
|---|---|---|
| DEN HAAG | 06-12-1988 | BRIGHENTI |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument